# EUROPEAN PATENT SPECIFICATION

(11) **EP 4 464 763 B1**
(45) Date of publication and mention of the grant of the patent: **25.03.2026**
(21) Application number: 24169925.5
(22) Date of filing: 12.04.2024
(51) Int. Cl.: C11D 3/22

(54) **DISSOLVABLE NANOCELLULOSE SHEET**
LÖSLICHE NANOCELLULOSEFOLIE
FEUILLE DE NANOCELLULOSE SOLUBLE

(30) Priority: 19.05.2023 US 202363467661 P; 07.03.2024 US 202418598693
(43) Date of publication of application: 20.11.2024
(73) Proprietor: Berkley Medical Resources, Inc., Smithfield, PA 15478 (US)
(72) Inventor: Tommarello, Domenic, Cranberry Township, 16066 (US); Berkley, Sherry, Kiawah Island, 29455 (US)
(74) Representative: Murgitroyd & Company

(56) References cited:
- US-A1- 2005 003 991
- US-A1- 2020 277 555
- US-A1- 2021 261 885
- US-A1- 2022 412 010
- DENG ZILONG ET AL: "Development, characterization, and validation of chitosan adsorbed cellulose nanofiber (CNF) films as water resistant and antibacterial food contact packaging", LWT- FOOD SCIENCE AND TECHNOLOGY, ACADEMIC PRESS, UNITED KINGDOM, vol. 83, 13 May 2017 (2017-05-13), pages 132 - 140, XP085069442, ISSN: 0023-6438, DOI: 10.1016/J.LWT.2017.05.013

## Description

### FIELD OF THE DISCLOSURE

The present disclosure relates to a dissolvable sheet. The dissolvable sheet composition is composed of a cellulose substate and an active ingredient composition. The present disclosure also relates to methods of manufacturing a dissolvable sheet from a liquid precursor formulation.

### BACKGROUND

The environmental safety of consumer products is a growing concern among customers and manufacturers alike. As the adverse environmental effects of certain ingredients in common household products become more available, the demand for biodegradable, eco-friendly, and sustainable products increases. In one example, many laundry products contain environmentally harmful chemicals such as polyvinyl alcohol (PVA). Some countries have regulations in place regarding the disposal of PVA products to prevent environmental pollution. For example, in the European Union, PVA is subject to the Waste Framework Directive, which sets out rules for waste management and disposal to protect human health and the environment. The Directive requires that PVA products be disposed of in a way that minimizes their impact on the environment, such as through composting or in dedicated waste treatment facilities. It is likely that other countries will adopt stricter regulations or bans on PVA as concerns rise about its environmental impact.

In addition, consumers now have their choice of several delivery systems for household products, e.g., laundry products and personal care products, such as bathing and hygiene aids, and industrial products, e.g., agricultural products. For example, laundry pods have recently become popular as an alternative to liquid detergent. These pods are small, self-contained packets that contain a pre-measured amount of detergent, which is often mixed with other fragrance additives or fabric softeners. However, laundry pods can sometimes leave residues on fabrics if the pod membrane does not fully disperse in the wash cycle. Additionally, the size, shape, and appearance of laundry pods is a potential a safety issue for young children who may be tempted to ingest them. Accordingly, there exists a need to provide an environmentally friendly substrate that facilitates delivery and dispersal of household and industrial ingredients. The present disclosure provides biodegradable sheet compositions to overcome the foregoing drawbacks and meet such needs.

US 2022/412010A and Deng Zilong et al: " Development, characterization, and validation of chitosan adsorbed cellulose nanofiber (CNF) films as water resistant and antibacterial food contact packaging", (LWT-Food Science and Technology, Academic Press, UK, vol. 83, 13 May 2017, pages 132-140), are useful for understanding the present invention.

### SUMMARY OF THE DISCLOSURE

The present disclosure provides a dissolvable sheet as defined in the claims. Methods for making and using the dissolvable sheets are also described.

Advantageously, the dissolvable sheet composition is capable of quickly and completely dispersing without leaving residue. In laundry applications, the sheet has good cleaning performance despite using less active ingredient compared to other laundry detergent formulations. The dissolvable sheet substrate may be derived from renewable sources and be free of environmentally damaging chemicals and is free of polyvinyl alcohol.

In some of any embodiments, the chemically modified version of cellulose is hydroxyethyl cellulose (HEC), hydroxypropyl cellulose (HPC), hydroxypropyl methylcellulose (HPMC), methyl cellulose, or carboxymethylcellulose sodium (NaCMC).

In some of any embodiments, the cellulose derivative is NaCMC, where the CMC is present in an amount of 1 to 15 wt.% relative to the total weight of the sheet. In some of any embodiments, the active ingredient composition includes at least one surfactant, an enzyme blend, a starch, a dispersion aid, a fragrance, a preservative, or a combination thereof.

In some of any embodiments, the enzyme blend includes at least one protease enzyme, at least one amylase enzyme, at least one pectate lyase enzyme, at least one mannanase enzyme, at least one lipase enzyme, or a combination thereof.

The dissolvable sheet includes 25 wt.% to 55 wt.% cellulose, 10 wt.% to 45 wt.% of a nonionic surfactant, 1 wt.% to 10 wt.% of an anionic surfactant, 1 wt.% to 15 wt.% of a cellulose derivative, 1 wt.% to 10 wt.% of glycerine, 0.1 wt.% to 8 wt.% of an enzyme blend, 0.5 wt.% to 8 wt.% of a starch, 0.01 wt.% to 0.5 wt.% of a preservative, and 1-20 wt.% water, relative to the total weight of the dissolvable sheet.

In some of any embodiments, the active ingredient composition is releasable from the sheet when exposed to water. In some of any embodiments, the sheet has a weight of about 1 gram to 8 grams, where the range is inclusive. In some of any embodiments, the sheet has a thickness of about 0.1 to 0.3 millimeters, where the range is inclusive.

In some of any embodiments, the nanocellulose substrate comprises a nanocellulose fiber which is naturally derived from softwood, hardwood, soy hulls, wheat straw, bagasse, sugar beet pulp, or a combination thereof.

The dissolvable sheets may be formed from liquid formulations, such as liquid precursors, also discussed herein

In some of any embodiments, the chemically modified version of cellulose is NaCMC, where NaCMC is present in an amount of 2.5 wt. % to 6 wt. % relative to the total weight of the liquid formulation.

In a third aspect, the present disclosure relates to methods of manufacturing dissolvable sheets as defined in the claims.

In some embodiments, a method of manufacturing a dissolvable sheet includes (i) processing the liquid formulation as defined herein through a roll coater onto a slip sheet, (ii) drying the liquid formulation by passing the slip sheet through a heater to form a dried sheet roll, and (iii) cutting the dried sheet roll into individual dissolvable sheets.

In some of any embodiments, the heater is a convection oven. In some of any embodiments, the slip sheet passes through the heater at a speed of from about 0.5 meters per minute to 1.5 meters per minute.

In some of any embodiments the liquid formulation is not heated above 49 °C (120°F) and the maximum internal temperature of the sheet roll does not exceed 49 °C (120°F).

### BRIEF DESCRIPTION OF THE DRAWINGS

FIG. 1A is an image of the inventive laundry sheet after a 5 minute dispersion cycle in 18°C (65°F) purified water, and FIG. 1B is an image of a National Brand PVA laundry sheet after a 5 minute dispersion cycle in 18°C (65°F) purified water.
FIG. 2A is a microscope image of the inventive laundry sheet surface at 8x magnification and FIG. 2B is a microscope image of a National Brand PVA laundry sheet surface at 8x magnification.
FIG. 3A is a microscope image of the inventive laundry sheet surface at 4x magnification and FIG. 3B is a microscope image of a National Brand PVA laundry sheet surface at 4x magnification.
FIG. 4A, 4B, 4C, 4D, 4E, 4F, and 4G are scanning electron microscope images of a coated National Brand PVA laundry sheet at various magnifications.
FIG. 5A, 5B, and 5C, are scanning electron microscope images of an uncoated National Brand PVA laundry sheet at various magnifications.
FIG. 6A, 6B, 6C, 6D, and 6E are scanning electron microscope images at various magnifications of a coated nanocellulose sheet including an exemplary active ingredient composition as disclosed herein.
FIG. 7A, 7B, and 7C are scanning electron microscope images at various magnifications of an uncoated dissolvable matrix as disclosed herein.
FIG. 8A is a scanning electron microscope image of a National Brand PVA laundry sheet and FIG. 8B is an elemental analysis spectrum of the indicated window in FIG. 8A.
FIG. 9A is a scanning electron microscope image of a disclosed inventive sheet, and FIG. 9B is an elemental analysis spectrum of the indicated window in FIG. 9A.
FIG. 10 is an elemental analysis overlay comparison between a disclosed inventive sheet and the National Brand PVA laundry sheet.
FIG. 11A is a scanning electron microscope image of a disclosed inventive sheet. FIG. 11B and FIG. 11C are elemental analysis spectra of two different areas indicated in FIG. 11A.

### DETAILED DESCRIPTION

### I. Definitions

Before the present compositions and methods are disclosed and described, it is to be understood that the compositions and methods are not limited to specific synthetic methods, specific components, or to particular compositions. It is also to be understood that the terminology used herein is for the purpose of describing particular embodiments only and is not intended to be limiting.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. When ranges are listed in the specification and in the claims, it is understood that all the numbers including decimals within the range are included whether specifically disclosed. For example, if the range is from 1 to 10, the range would include every number within the range, such as 1; 1.1; 1.2; 1.3; 1.4; 1.5; 1.6; 1.7; 1.8; 1.9; 2; 2.1; 2.2; 2.3; 2.4; 2.5; 2.6; 2.7; 2.8; 2.9; 3; 3.1; 3.2; 3.3; 3.4; 3.5; 3.6; 3.7; 3.8; 3.9; 4; 4.1; 4.2; 4.3; 4.4; 4.5; 4.6; 4.7; 4.8; 4.9; 5; 5.1; 5.2; 5.3; 5.4; 5.5; 5.6; 5.7; 5.8; 5.9; 6; 6.1; 6.2; 6.3; 6.4; 6.5; 6.6; 6.7; 6.8; 6.9; 7; 7.1; 7.2; 7.3; 7.4; 7.5; 7.6; 7.7; 7.8; 7.9; 8; 8.1; 8.2; 8.3; 8.4; 8.5; 8.6; 8.7; 8.8; 8.9; 9; 9.1; 9.2; 9.3; 9.4; 9.5; 9.6; 9.7; 9.8; 9.9, and 10.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," or "derived from" and is not intended to exclude, for example, other additives, components, integers or steps. "Exemplary" means "an example of" and is not intended to convey an indication of a preferred or ideal embodiment. "Such as" is not used in a restrictive sense, but for explanatory purposes.

As used herein, the term "active ingredient" or "active agent" refers to any substance that produces a desired chemical, physical, or biological effect, wherein the substance is capable of producing this effect independent of its association with a substrate or carrier material.

With respect to the terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error or minor adjustments made to optimize performance, for example. In the event it is determined that individuals having ordinary skill in the relevant arts would not readily ascertain values for such reasonably small differences, the terms "about" and "approximately" can be understood to mean plus or minus 10% of the stated value.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific embodiment or combination of embodiments of the disclosed methods.

### I. Dissolvable Matrix Compositions

Provided herein are dissolvable matrix compositions composed of a nanocellulose substrate and a cellulose derivative. Disclosed dissolvable matrix compositions may also contain an active ingredient composition. In other embodiments not according to the invention, disclosed dissolvable matrix compositions may not contain an active ingredient composition and instead consist essentially of a cellulose substrate and a cellulose derivative. Such compositions are free of PVA, which is typically used as a binder, stabilizer, or to improve flow. In the absence of PVA, which has been recognized as an environmental pollutant, the dissolvable matrix compositions provided herein can physically and/or chemically support active agents useful in household or industrial application. Such dissolvable substrate compositions advantageously serve as vehicles for carrying active agents to their site of action, facilitating their intended use, while also exhibiting dispersion properties for releasing active ingredient compositions, such as upon contact with a sufficient volume of liquid.

Disclosed dissolvable matrix compositions can be redispersed in water or other aqueous redispersion solvents. Such dispersal or dissolution aids the release of any active agents incorporated in the matrix into solution. In some embodiments, the matrix serves to convey the active agent to a site of its activity or dispense active agents in a desired environment over a desired time period. As used herein, the terms "dissolvable," "dispersible," "redispersible," and the like refer to a process by which the dissolvable matrix is suspended in a fluid medium, whether aqueous or non-aqueous, so that there is a substantially complete dissolution of the dissolvable matrix into its component elements. In embodiments, aqueous resuspending fluids can be used. In other embodiments, non-aqueous resuspending fluids can be used, such as fluids having hydrophobic properties or amphiphilic properties.

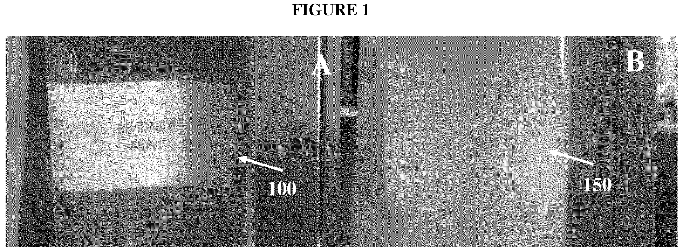

Disclosed dissolvable matrix compositions can be dispersed in a sufficient volume of liquid, e.g., a volume of water sufficient to facilitate redispersion of the dissolvable matrix. Agitation can be applied to facilitate dispersion, e.g., shaking, rotating, or using a magnetic stir bar to stir a solution containing a disclosed dissolvable matrix. The minimum volume of liquid necessary for redispersing a disclosed dissolvable matrix may range from 0.5L to 4L, 0.5L to 3L, 0.5L to 2L, 0.75L to 4L, 0.75L to 3L, 0.75L to 2L, 1L to 4L, 1L to 3L, or 1L to 2L, wherein each range is inclusive. The temperature of the liquid may range from 15°C to 65°C, 25°C to 65°C, 45°C to 65°C, 55°C to 65°C, 15°C to 55°C, 25°C to 55°C, 35°C to 55°C, 45°C to 55°C, 15°C to 45°C, 25°C to 45°C, or 35°C to 45°C, wherein each range is inclusive. In some examples, disclosed dissolvable matrix compositions may substantially disperse, such as dissolve into a clear solution free from visible intact particles, in as little as 3 minutes, 5 minutes, 7 minutes, or 10 minutes. In other examples, disclosed dissolvable matrix compositions may substantially disperse, such as dissolve into a clear solution free from visible intact particles, after a period of 15 minutes or more, 30 minutes or more, 45 minutes or more, or 60 or more. In yet other examples, disclosed dissolvable matrix compositions may substantially disperse, such as dissolve into a clear solution free from visible intact particles, after a period of day(s), e.g., 1 day or more 3 days or more, 7 days or more, 25 days or more, or 60 days or more, week(s), e.g., 1 week or more, 2 weeks or more, 3 weeks or more, or 4 weeks or more, or month(s), e.g.,1 month or more, 3 months or more, 6 months or more, 9 months or more, or 12 months or more. In one representative example Figure 1 shows substantial dispersion of an exemplary disclosed dissolvable matrix upon exposure to about 1600 ml of purified water at a temperature of about 18°C after 5 minutes of agitation by a magnetic stir bar. As shown in Figure 1A, the readable print 100 placed behind the beaker containing the dissolved matrix is visible. In contrast, the cloudiness of a National Brand PVA laundry sheet after a 5 minute dispersion cycle in 65°F purified water prevents the readable print from being visible 150.

In some embodiments, redispersion results in a suspension including a population of particles with a non-uniform distribution of various parameters, including size and shape. Some components may not fully dissolve and instead disperse as larger aggregates. In some examples, dispersibility can be observed by visual observation. For example, the presence of larger intact particles of a comparator matrix following contact with a sufficient volume of liquid indicates relatively poor dispersibility versus smaller intact particles or the absence of the same after exposing a disclosed matrix exposed to the same volume of liquid under the same conditions. The turbidity of a solution can be compared to a standard, e.g., distilled water, to qualitatively assess clarity and dispersibility. In other examples, the turbidity of a solution containing a dispersed matrix can be assessed using a turbidity meter or UV-vis spectroscopy.

In additional examples dynamic light scattering (DLS) can be used to determine the size distribution of particles in a solution to aid in determining the extent of dissolution. In some examples, redispersion results in a suspension of the dissolvable matrix including suspended particles have an aspect ratio of greater than 1, 5, 10, 15, or 20. In some embodiments, the resuspended particles have an aspect ratio between about 10 and about 300, between about 10 and about 150, between about 10 and about 200, between about 50 and about 150, between about 50 and about 100, between about 75 and about 125, between about 25 and about 75, wherein each range is inclusive. Aspect ratio can be determined using methods available to one of skill in the art, including microscopy and image analysis software.

In some embodiments, disclosed dissolvable matrix compositions can be formulated, shaped, or molded into a single layer or multi-layer sheets, as required by any product's specific needs. For example, dissolvable matrix compositions in sheet form can be laundry sheets, in-scent booster sheets, odor neutralizer sheets, fabric softener sheets, all-purpose cleaning sheets, toilet bowl sheets, bathroom cleaner sheets, hand soap sheets, skin and makeup cleansers, dishwashing sheets, or auto dishwash sheets.

Such articles can be formed by first providing a liquid formulation containing raw materials dissolved or dispersed in water, then shaping the wet mix into the desired form of a sheet. Drying is carried out either simultaneously with the shaping step, or it can be carried out subsequently to remove water and form a dried dissolvable sheet. In preferred embodiments, disclosed matrix compositions are molded, cut, or otherwise oriented as a sheet. For example, the matrix composition may be formed into sheets that have detergent and/or laundry agents disposed within the matrix interstices. The matrix may be delivered into a washing machine and allowed to come into contact with water, allowing the laundry agents to be delivered and the matrix to be dissolved thereby facilitating dispersal of its components.

The dissolvable matrix compositions of the disclosure may have a relatively smaller total weight compared to other household products, industrial compositions, or sheet articles. For example, a dissolvable sheet may have a total weight of about 1 to 5 g, 1 to 10 g, 1 to 15 g, 2 to 8 g, 2 to 6g, 3 to 6 g, 5 to 10 g, or 5 to 15 g wherein each range is inclusive. In some examples, the total weight of a dissolvable sheet may be as low as about 0.1 g, 0.5 g, 1 g, 1.5 g, 2 g, 2.5 g, 3 g, 3.5 g, 4 g, 4.5 g, 5 g, 5.5 g, or as high as 6 g, 7 g, 8 g, 9 g, 10 g, or within any range encompassed by any two of the foregoing values as endpoints.

Disclosed dissolvable matrix compositions may have thinness which also contributes to its relatively smaller total weight. For example, a dissolvable sheet may have a thickness as low as 0.1 mm, 0.11 mm, 0.12 mm, 0.13 mm, 0.14 mm, 0.15 mm, 0.16 mm, 0.17 mm, 0.18 mm, 0.19 mm, 0.20 mm, 0.21 mm, 0.22 mm, 0.23 mm, or as high as 0.24 mm, 0.25 mm, 0.26 mm, 0.27 mm, 0.28 mm, 0.29 mm, 0.30 mm, 0.31 mm, 0.32 mm, 0.33 mm, 0.34 mm, 0.35 mm, 0.36 mm, 0.37 mm, 0.38 mm, 0.39 mm, 0.40 mm, or within any range encompassed by any two of the foregoing values as endpoints. For example, the sheet may be from 0.1 to 0.3 mm thick.

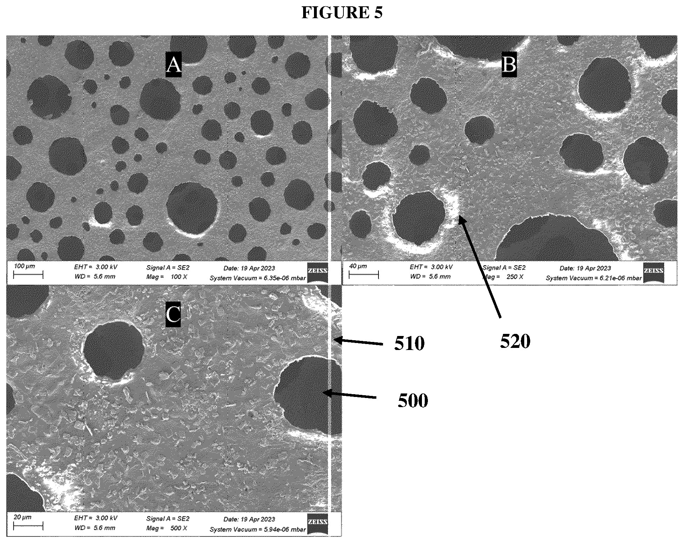

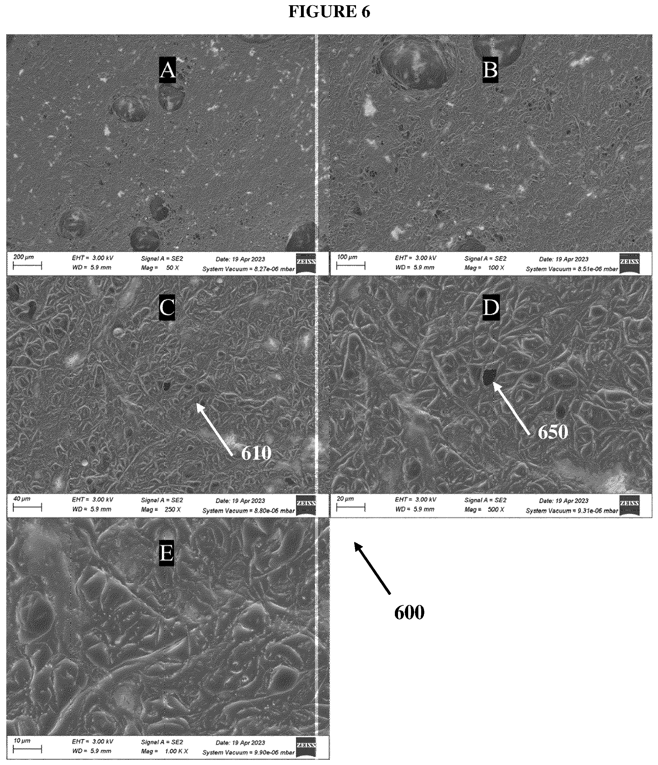

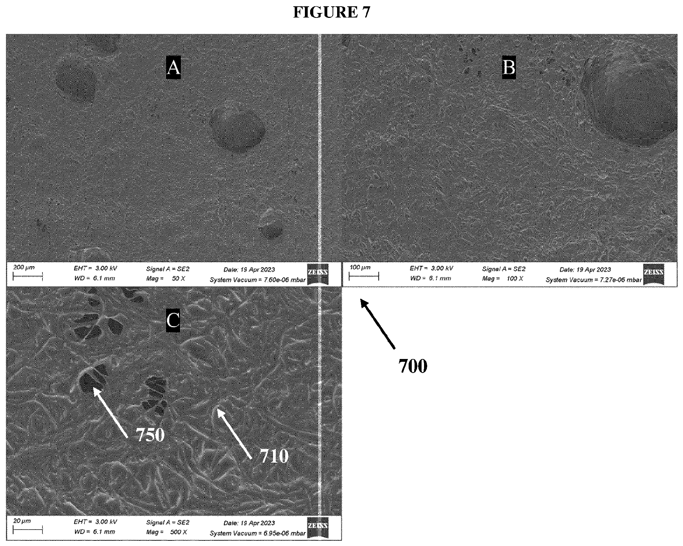

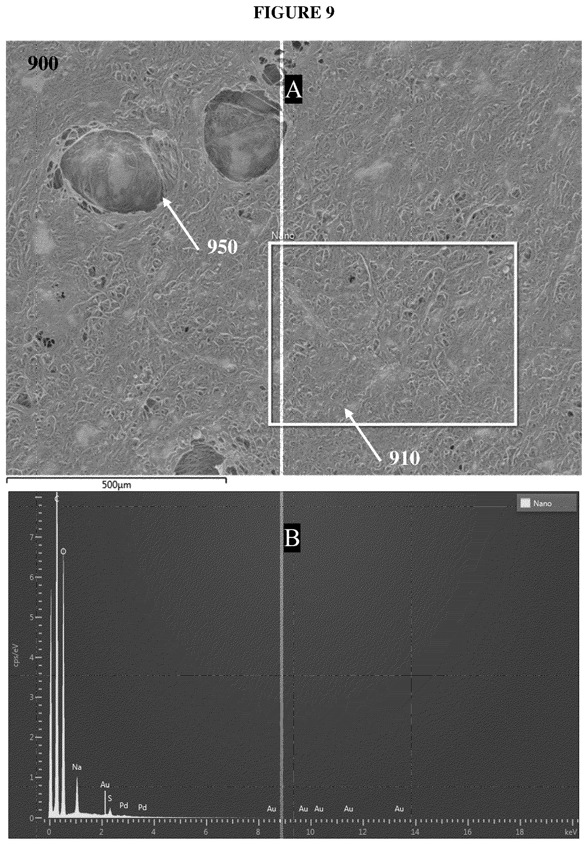

Dissolvable matrix compositions as disclosed herein can be formed into highly porous three-dimensional nanoscale networks capable of holding active ingredients within their interstices. For comparison, Figs. 5A-C show electron absorption in areas that are not uniform in conductivity 520. Figs. 6, 7, and 9 provide scanning electron microscope (SEM) images of a coated exemplary dissolvable sheet composition including a nanocellulose substrate with active ingredients 600 or an uncoated exemplary dissolvable sheet composition including a nanocellulose substrate without active ingredients 700. The same features indicating non-uniform conductivity are not present in Figs. 7A-7C, which has more uniform conductivity throughout. Without being bound to theory, it is believed that the uniform, randomly laid fiber strands of the nanocellulose sample contribute to the improved conductivity. The coated sheet sample images were made to assure sufficient conductivity for the SEM such that a high quality image could be obtained. Paper-type products such as nanocellulose are generally nonconductive. The uncoated sheet sample images were made after the finding that there was plenty of conductivity present to obtain a high quality image.

### A. Cellulose Substrate

Disclosed dissolvable matrix compositions contain cellulose. Cellulose, the main building block for wood and plant fibers, is an abundant resource for the paper, textile, and chemical industries. It is a high molecular weight homopolymer of 1,4-linked β-D-glucopyranose units in which each unit is rotated 180° with respect to adjacent units. The monomeric glucopyranose units each contain three hydroxyl groups, which present themselves on alternating opposite sides of the polymer because of the rotational pattern of their linear arrangement within the polymer. The alternating orientation of the hydroxyl groups along the length of the cellulose molecule allows one strand of cellulose to form hydrogen bonds readily with adjacent strands of the polymer. These hydrogen bonds permit the formation of multistrand composites that are stable, strong, and tightly cohesive. The structure and applications of cellulose, in particular nanocellulose, have been described, e.g., by Trache et al., Front. Chem. Sec. Polymer Chemistry, 2020(8). These advantageous properties of cellulose make it a suitable base material for a variety of applications.

The nanocellulose substrate of the present disclosure may be formed by processing raw cellulose sources using mechanical techniques and optional chemical treatments to extract the component cellulose nanomaterials. For example, mechanical treatments such as high-pressure homogenization, micro-fluidization, super-grinding, cryo-crushing, steam explosion, refining, and ultrasonication can be used for disintegrating the cellulose source materials to yield their component nanocellulose elements. Optional chemical treatments may be used to optimize the surface chemistry of nanocellulose elements. These elements can be pretreated with short amines or positive oligomeric species to mitigate ionic forces between the nanocellulose elements. Examples of chemical treatment agents may include ethylenediamine, o-phenylenediamine, diethylenetriamine, tetraethylenepentamine, 1,3 - diaminopentane, ethanolamine, triethynolamine, melamine, and ethylenediaminetetraacetic acid (EDTA).

Suitable raw sources of cellulosic material for disclosed compositions include softwood, hardwood, soy hulls, wheat straw, bagasse, sugar beet pulp, and the like. Further exemplary sources of cellulosic material, including nanocellulose, is described by Trache et al., Front. Chem. Sec. Polymer Chemistry, 2020(8). Additionally, extraction of nanocellulose has been described, e.g., by Shen et al., Carbohydr Polym. 2022 Aug 15:290:119462, Jasmani & Adnan, Carbohydr Polym. 2017 Apr 1:161:166-171, and Hachaichi et al., Materials (Basel). 2021 Sep 15;14(18):5313. Cellulose fibers are available for purchase by a variety of vendors, including Sigma-Aldrich, Grainger Industrial Supply, Nanografi Nano Technology, The University of Maine, CeulluForce Inc., Sappi Europe SA, Kruger Inc., and Nippon Paper Industries Co., Ltd. among others.

In some embodiments, disclosed dissolvable matrix compositions include a nanocellulose substrate including fibrillated cellulose. The nanocellulose substrate can include microfibrillated cellulose, cellulose nanofibers, cellulose nanofibrils, bacterial nanocellulose, or a combination thereof. Herein, the term "nanocellulose" and "nanocellulose substrate" are used interchangeably to describe nanoscale or nano-structured cellulose, which includes cellulose nanocrystals, cellulose nanofibers, and combinations thereof.

As noted, a nanocellulosic substrate as described herein can include microfibrillated cellulose and nanocellulose, which may include cellulose nanofibers (nanofibrillated cellulose), cellulose nanofibrils, bacterial nanocellulose, or a combination thereof. While the terms "nanocellulose" and "nanocellulose substrate" are used interchangeably to describe nanoscale or nano-structured cellulose, reference to a nanocellulose material does not exclude the presence of cellulose fiber that exceeds nanoscale. For example, a nanocellulose substrate may also include micron-scale cellulose fibers.

The size of fibers in a cellulose substrate can be represented by particle size populations. For example, where "D" refers to particle size distribution, "D10" represents that 10% of particles in a given population are smaller than a specific size; "D50" represents that 50% of particles in a given population are smaller than a specific size; and "D90" represents that 90% of particles in a given population are smaller than a specific size.

In some examples, disclosed dissolvable matrix compositions include a cellulose substrate with a D10 value ranging from 1 µm to 35 µm, 5 µm to 35 µm, 10 µm to 35 µm, 25 µm to 35 µm, 1 µm to 25 µm, 5 µm to 25 µm, 10 µm to 25 µm, or 15 µm to 25 µm; a D50 value ranging from 50 µm to 150 µm, 75 µm to 150 µm, 100 µm to 150 µm, 50 µm to 125 µm, 75 µm to 125 µm, 100 µm to 125 µm, 50 µm to105 µm, 75 µm to 105 µm, or 100 µm to 105 µm; and a D90 value ranging from 350 µm to 950 µm, 550 µm to 950 µm, 750 µm to 950 µm, 250 µm to 750 µm, 450 µm to 750 µm, 600 µm to 750 µm, 400 µm to 600 µm, 450 µm to 600 µm, 500 µm to 600 µm, or 550 µm to 600 µm, wherein each range is inclusive.

In some embodiments, disclosed dissolvable matrix compositions may include a cellulose substrate in an amount as low as 20 wt.%, 21 wt.%, 22 wt.%, 23 wt.%, 24 wt.%, 25 wt.%, 26 wt.%, 27 wt.%, 28 wt.%, 29 wt.%, 30 wt.%, 31 wt.%, 32 wt.%, 33 wt.%, 34 wt.%, 35 wt.%, 36 wt.%, 37 wt.%, 38 wt.%, 39 wt.%, 40 wt.%, 41 wt.%, 42 wt.%, 43 wt.%, 44 wt.%, 45 wt.%, 46 wt.%, 47 wt.%, 48 wt.%, 49 wt.%, or 50 wt.% relative to the total weight of the dissolvable matrix composition, or within any range encompassed by any two of the foregoing values as endpoints. For example, the cellulose may be present in an amount of from 30 wt.% to 60 wt.%, from 30 wt.% to 50 wt.%, from 30 wt.% to 40 wt.%, from 31 wt.% to 39 wt.%, from 32 wt.% to 38 wt.%, from 33 wt.% to 37 wt.%, or from 34 wt.% to 36 wt.% relative to the total weight of the dissolvable matrix composition, wherein each range is inclusive.

In yet other embodiments, disclosed dissolvable matrix compositions may include a cellulose substrate in an amount of 10 wt.% to 70 wt.%, 15 wt.% to 85 wt.%, 25 wt.% to 90 wt.%, 10 wt.% to 75 wt.%, 15 wt.% to 80 wt.%, 20 wt.% to 75 wt.%, 10 wt.% to 60 wt.%, 15 wt.% to 65 wt.%, 20 wt.% to 65 wt.%,, 25 wt.% to 65 wt.%, 30 wt.% to 65 wt.%, or 35 wt.% to 65 wt.% relative to the total weight of the dissolvable matrix composition, wherein each range is inclusive.

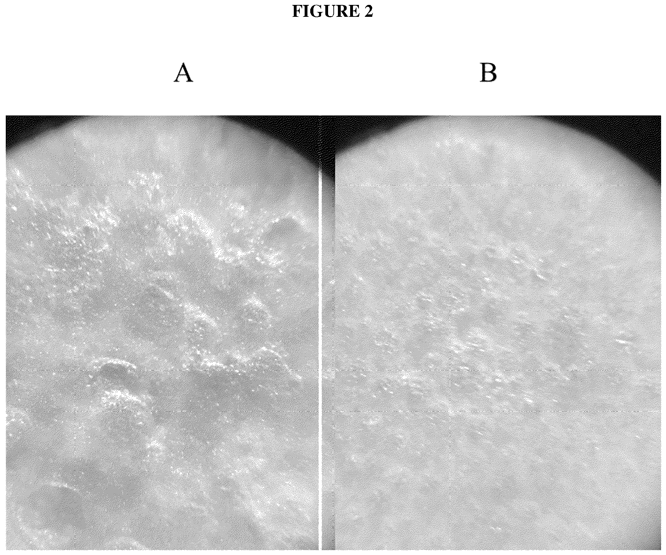

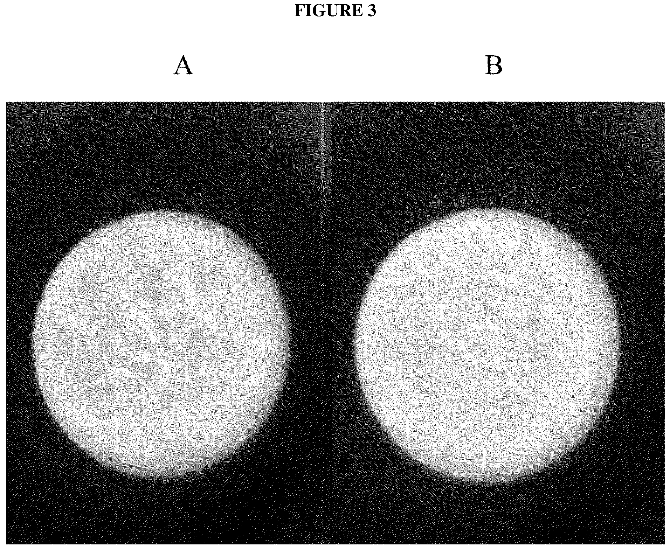

In certain embodiments, such dissolvable matrix compositions including a nanocellulose substrate, have a non-woven structure, such as a non-woven web of cellulosic material, such as a nanocellulose matrix. The fibers of nanocellulose substrates, such as nanocellulose fibers, can be bonded to each other by mechanical, hydro-mechanical, thermal, or chemical means. However, the term "non-woven" indicates that the fibrils have not been interlaced, such as thread or yarn. Rather the non-woven structure includes randomly oriented fibers, which contrast with the grid-like structure of woven thread or yarn. For example, Figures 2 and 3 show optical microscope images of a representative non-woven substrate. The image shows no repeating units or weave pattern, consistent with the described non-woven web structure.

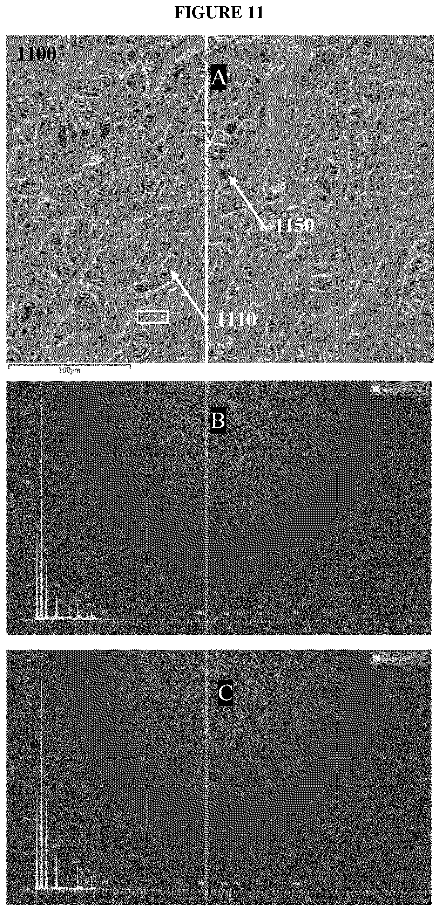

Figures 6, 7, 9, and 11, for example, show the non-woven microstructure of exemplary matrix compositions. See 610, 710, 910, and 1100. The network of fibers form pores of varying sizes. See 650, 750, 950, and 1150. In some embodiments, the pores have a maximum length, as measured from the greatest end-to-end distance of about 100 µm to 950 µm, 150 µm to 850 µm, 200 µm to 750 µm, 250 µm to 650 µm, 300 µm to 550 µm, or 400 to 550 µm, wherein each range is inclusive. In some embodiments, the pores have a maximum length, as measured from the greatest end-to-end distance of about 1 µm to 500 µm, 1 µm to 250 µm, 1 µm to 200 µm, 5 µm to 300 µm, 5 µm to 250 µm, 5 µm to 200 µm, 10 µm to 300 µm, 10 µm to 250 µm, or 10 µm to 200 µm, wherein each range is inclusive. In other embodiments, the pores have a maximum length, as measured from the greatest end-to-end distance of about 1 µm to 30 µm, 1 µm to 25 µm 1 µm to 20 µm, 5 µm to 30 µm, 5 µm to 25 µm, 5 µm to 20 µm, 10 µm to 30 µm, 10 µm to 25 µm, or 10 µm to 20 µm, wherein each range is inclusive. In some embodiments, the pores have a maximum length, as measured from the greatest end-to-end distance of about 10 µm, 11 µm, 12 µm, 13 µm, 14 µm, 15 µm, 16 µm, 17 µm, 18 µm, 19 µm, 20 µm, 21 µm, 22 µm, 23 µm, 24 µm, or 25 µm.

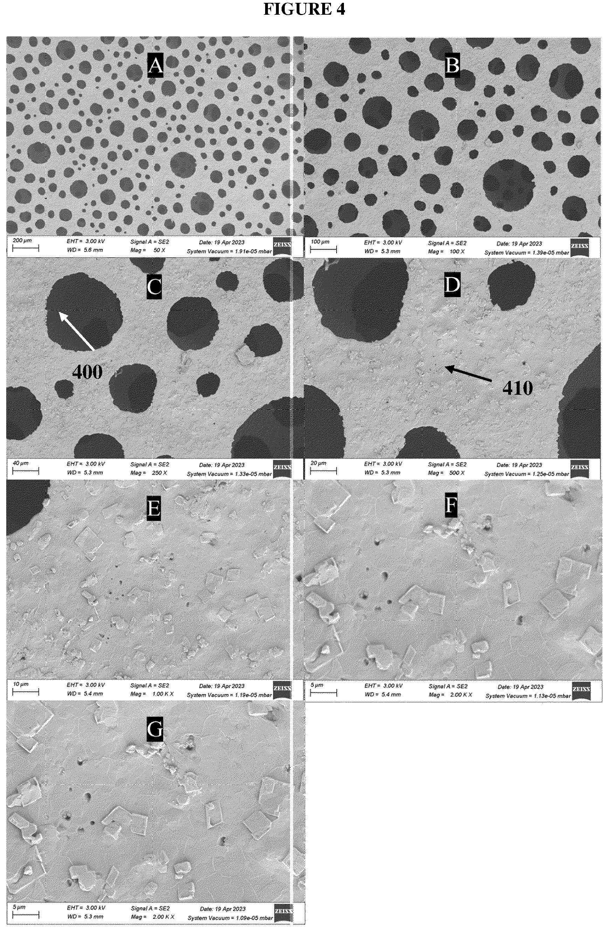

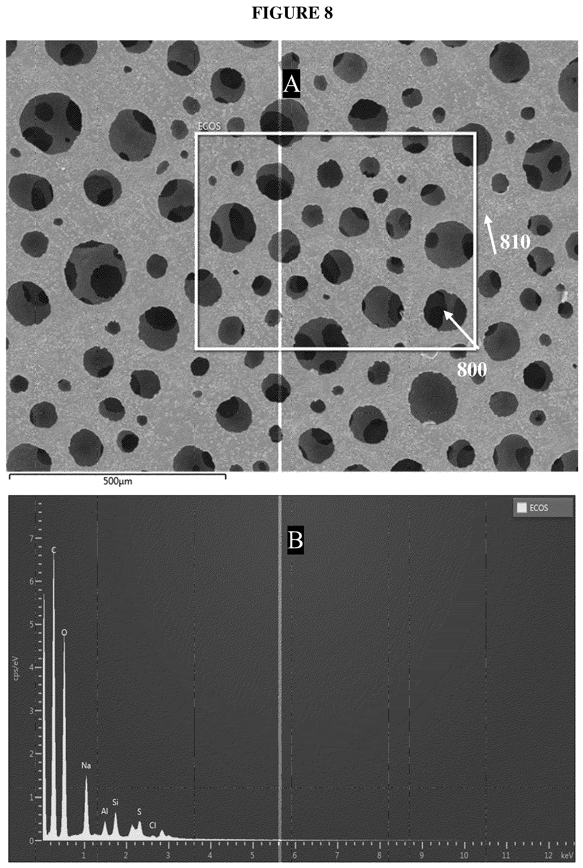

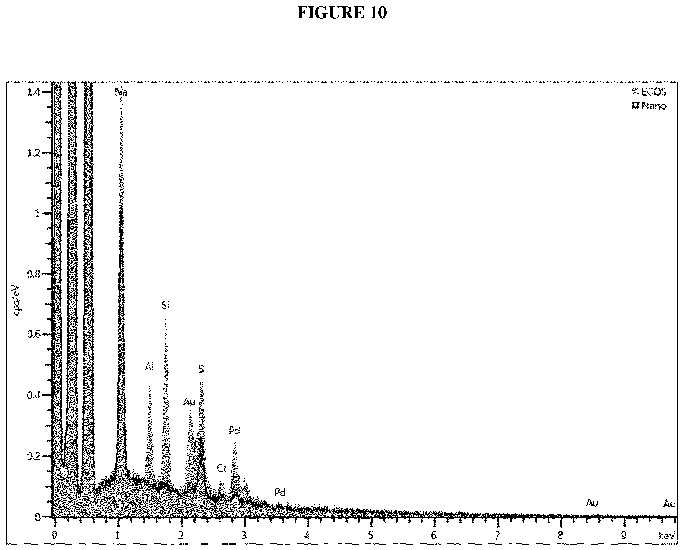

For context, Figures 4, 5, and 8 provide SEM images of a National Brand PVA laundry sheet. See 410, 510, and 810. The pore structure is more uniform, and the shapes and sizes of the individual pores are more consistent. See 400, 500, and 800. The elemental analysis spectra provided in Figs. 8B, 9B, and 10 show measured carbon at higher than 12 counts per second per energy supplied for the nanocellulose sheet while the National Brand PVA sheet measured below 7 counts per second per energy supplied. This result indicates that the organic content of the dissolvable sheet composition according to the present disclosure is higher.

### B. Cellulose Derivatives

Disclosed matrix compositions include chemically modified forms of cellulose, being either a cellulose ether or a cellulose ester. Although cellulose is insoluble in water and most common organic solvents, such cellulose derivatives are soluble in a wide range of solvents. For example, cellulose acetate is soluble in acetone, and other organic solvents, while methylcellulose, ethyl hydroxyethyl cellulose, and sodium carboxymethyl cellulose (NaCMC) are water soluble. Cellulose derivatives can be dissolved either at a molecular or colloidal level. See, e.g., Schulz et al., Macromol. Chem. Phys. 2000;201:2008-2022 and Burchard, Cellulose. 2003;10:213-225.

The surface chemistry of nanocellulose varies, depending on both the raw source of the cellulosic material (e.g., softwood, hardwood, soy hulls, wheat straw, bagasse, sugar beet pulp, and the like) and the processing technique implemented (e.g., Kraft vs Sunburst). Chemical treatments such as carboxymethylation, oxidation, and sulfonation can be implemented to create permanent anionic charges on the surface of nanocellulose. In one example, the water-soluble derivative of cellulose NaCMC is an anionic polysaccharide that is formed by reacting cellulose with sodium hydroxide and chloroacetic acid. In other examples, nanocellulose can be pretreated with short amines or positive oligomeric species to mitigate ionic forces. Such pre-treatment agents include but are not limited to ethylene diamine, o-phenylenediamine, diethylenetriamine, tetraethylenepentamine, 1,3-diaminopentane, ethanolamine, triethynolamine, melamine, and ethylenediaminetetraacetic acid (EDTA), and other pre-treatment agents familiar to those having ordinary skill in the art. Commercial sources of cellulose derivatives include Enartis, Sigma-Aldrich, Nouryon, Shin-Etsu Chemical, and Industrial Cellulosics, among others.

Exemplary cellulose ethers for inclusion in disclosed nanocellulose substrates or precursor liquid formulations thereof include carboxymethylcellulose, ethyl cellulose (ether cellulose), methyl cellulose, hydroxylethyl cellulose (HPMC), hydroxypropyl cellulose (HPC), hydroxypropylmethyl cellulose (HPMC), and ethylhydroxyethyl cellulose. Exemplary cellulose esters for inclusion in disclosed nanocellulose substrates or precursor liquid formulations thereof include cellulose acetate and cellulose acetate phthalate. In some examples, the cellulose ether or cellulose ester has a degree of substitution ranging from 0.5 to 1.

In some embodiments, disclosed dissolvable nanocellulose substrates and liquid precursor formulations thereof include the sodium salt of carboxymethylcellulose (NaCMC). In the absence of polyvinyl alcohol (PVA), the viscosity of liquid precursor formulations and the dispersibility of manufactured sheets were unexpectedly dependent upon the amount of NaCMC included in the liquid composition, as further described in Example 2 of the present disclosure. NaCMC included in disclosed nanocellulose substrates or precursor liquid formulations thereof can have low viscosity (0.05-0.2 Pa.s (50-200 cP), 4 % in H₂O at 25 °C), medium viscosity (0.4-0.8 Pa.s (400-800 cP), 2 % in H₂O at 25°C), or high viscosity (1.5-3 Pa.s (1500-3000 cP), 1 % in H₂O at 25°C). Viscosity is both concentration and temperature dependent. As temperature increases, viscosity is expected to decrease, and as concentration increases, viscosity is also expected to increase.

Disclosed dissolvable nanocellulose substrates and liquid precursor formulations thereof may include sodium CMC having a degree of substitution ranging from 0.6 to 1. The degree of substitution is the average number of carboxymethyl groups substituted per monomer unit, ranging from 0 to 3, with the remaining R = H. The structure and effects of the degree of substitution of sodium CMC has been described, e.g., Lopez et al., J Polym Sci B Polym Phys. 2015 Apr 1; 53(7): 492-501.

The sheet may comprise a cellulose derivative in an amount as low as 1 wt.%. 2 wt.%. 3 wt.%. 4 wt.%. 5 wt.%. 6 wt.%, 7 wt.%, 8 wt.%, 9 wt.%, 10 wt.%, or as high as 11 wt.%, 12 wt.%, 13 wt.%, 14 wt.%, 15 wt.%, or within any range encompassed by any two of the foregoing values as endpoints. For example, the sheet may comprise carboxymethylcellulose, such as NaCMC, in an amount of from 4 wt.% to 9 wt.%, 5 wt.%.to 15 wt.%., from 5 wt.% to 8 wt.% or from 6 wt.% to 7 wt.%.

### C. Active Ingredient Compositions

In some embodiments, disclosed dissolvable nanocellulose substrates include active ingredients, which can be supported by the nanocellulose matrix, embedded in it, attached to it, or otherwise associated with it. The active ingredients may be added to the liquid precursor formulation during the production of the dissolvable sheet. Alternatively, active agents may be sprayed, coated onto, or otherwise associated with a disclosed dissolvable matrix. The matrix, such as the non-woven cellulose web including a cellulose derivative, can support the inclusion of active agents having their own properties but that are intended to modify the intrinsic properties of the matrix itself, such as ingredients in consumer products, e.g., laundry products, personal hygiene products, and toiletries, as well as industrial products, including agricultural products.

In some embodiments, the dissolvable nanocellulose substrate may include an active ingredient such as a cleaning product, a laundry detergent, a surfactant, a dishwasher soap, a hand soap, a skin or makeup cleanser, s, a fabric softener, an agricultural composition, a dermatological composition (for example a moisturizer), a pharmaceutical composition, a cosmetic composition, or a combination of any of the foregoing. In some embodiments, the active ingredient composition may include an enzyme blend, a fragrance, odor neutralizer, a preservative, or a combination thereof. The active ingredients may be supported by the nanocellulose substrate, embedded in it, attached to it, or otherwise associated with it. In certain embodiments, the nanocellulose substrate can support the inclusion of active ingredients having their own properties intended to modify the intrinsic properties of the nanocellulose itself, such as pigments, dyes, or other agents which may impart their properties to the final formed article.

Upon contact with a sufficient amount of water, the nanocellulose substrate may disperse and release the active ingredients into solution. The nanocellulose substrate provides a matrix that suspends the active ingredients in solid form. When the nanocellulose substrate contacts a sufficient volume of water and dissolves, the active ingredients are released into the water. Advantageously, in certain embodiments of the present invention, the nanocellulose substrate serves as an effective alternative to comparable sheets containing PVA.

### i. Laundry Active Ingredients

In some embodiments, disclosed dissolvable sheets contain an active ingredient composition that includes at least one laundry active ingredient. Laundry related active ingredients include but aren't limited to detergents, surfactants, enzymes, detergent enzymes, starches, bleaches, fabric softeners, emulsifiers, scent boosters, static reducers, stain removal agents, anti-redeposition agent, degreasing agents, bleaching agents, bleach catalysts, whitening agents, fluorescent whitening agents, brighteners, dye transfer inhibiting agents, chlorine scavengers, chelating agents, corrosion inhibitors, opacifiers, suds suppressors, suds boosters, conditioning agents, colorants, dyes, fabric enhancing polymers, pH adjusters, buffering agents, antibacterials, antimicrobials, preservatives, malodor control agents, and fragrances or perfumes.

In some embodiments, the active ingredient composition includes a surfactant. Surfactants are molecules with surface-active properties that lower the surface tension or interfacial tension between two phases, such as two liquids. Often, surfactants are amphiphilic molecules with a relatively water-insoluble hydrophobic "tail" group and a relatively water-soluble hydrophilic "head" group. In systems comprising relatively polar and relatively non-polar components the hydrophobic tail preferentially interacts with the relatively non-polar component(s), while the hydrophilic head preferentially interacts with the relatively polar component(s). The surfactant may be uncharged, zwitterionic, cationic, or anionic. Although in principle any surfactant class (e.g., cationic, anionic, nonionic, amphoteric) is suitable, it is possible that an active ingredient composition may include a combination of two or more surfactants from two or more surfactant classes.

In some embodiments, the active ingredient composition includes a nonionic surfactant. Non-limiting examples of a nonionic surfactant include fatty monoglyceride ethoxylates, block copolymers, ethoxylated amines, alkyl amine oxides, mono or poly saccharide alkyl ethers, propoxylated and ethoxylated fatty alcohols, fatty acids, and alkyl phenols, ether terminated ethoxylates of fatty acid and fatty alcohols, fatty acid glucamides, alkyl glucosides, alkyl polyglucosides, and mixtures thereof. Additional nonionic surfactants include lauramine oxide and sodium laurylglucoside.

In some embodiments, the active ingredient composition includes a nonionic renewable surfactant, including, e.g., C8-C18 alkyl ethoxylates, such as, NEODOL^{®} nonionic surfactants; C6-C12 alkyl phenol alkoxylates where the alkoxylate units may be ethyleneoxy units, propyleneoxy units, or a mixture thereof; C12-C18 alcohol and C6-C12 alkyl phenol condensates with ethylene oxide/propylene oxide block polymers such as Pluronic^{®}; C14-C22 mid-chain branched alcohols, BA; C14-C22 mid-chain branched alkyl alkoxylates, BAEX, wherein x is from 1 to 30; and ether capped poly(oxyalkylated) alcohol surfactants. Other suitable renewable nonionic detersive surfactants include alkylpolysaccharides, such as alkylpolyglycosides, and methyl ester ethoxylates.

Additional non-limiting examples of nonionic surfactants include various condensation products. For example, the condensation product from a mixture of about one mole of a fatty alcohol or fatty acid and about five to about 10 moles of ethylene oxide can be used, such products are referred to herein as fatty alcohol ethoxylates and fatty acid ethoxylates, respectively. The fatty alcohol or fatty acid can be saturated or unsaturated while the chain can be straight or branched. In addition, the chain can contain from ten to twenty carbon atoms. Commercial products representative of alcohol ethoxylates include BIO-SOFT^{®} EC600 series products including BIO-SOFT^{®} EC600 and BIO-SOFT^{®} EC690 (lauryl alcohol ethoxylate, poe-7).

In some embodiments, the active ingredient composition includes an anionic surfactant. Suitable additional anionic surfactants include petroleum-derived alkoxylated alkyl sulfates (e.g., petroleum-derived ethoxylated alkyl sulfate surfactants), non-alkoxylated alkyl sulfates, and sulfonic detersive surfactants, e.g., alkyl benzene sulfonates.

Examples of non-alkoxylated, e.g., non-ethoxylated, alkyl sulfate surfactants include those produced by the sulfation of higher C8-C20 synthetic alcohols. In some examples, primary alkyl sulfate surfactants have the general formula: ROSO3 -M+, wherein R is a C8-C20 hydrocarbyl group, which may be straight or branched, and M is a water-solubilizing cation. In some examples, R is a C10-C15 alkyl, and M is an alkali metal.

Other useful anionic surfactants include the alkali metal salts of alkyl benzene sulfonates, in which the alkyl group contains from about 9 to about 15 carbon atoms, in straight chain (linear) or branched chain configuration. In some examples, the alkyl group is linear. Such linear alkylbenzene sulfonates are known as "LAS." In other examples, the linear alkylbenzene sulfonate may have an average number of carbon atoms in the alkyl group of from about 11 to 14. In a specific example, the linear straight chain alkyl benzene sulfonates may have an average number of carbon atoms in the alkyl group of about 11.8 carbon atoms, which may be abbreviated as C11.8 LAS. LAS may be derived from natural materials, including bioparaffin, natural alcohols and esters.

Suitable alkyl benzene sulfonate (LAS) may be obtained, by sulfonating commercially available linear alkyl benzene (LAB); suitable LAB includes low 2-phenyl LAB, such as those supplied by Sasol under the tradename Isochem^{®} or those supplied by Petresa under the tradename Petrelab^{®}, other suitable LAB include high 2-phenyl LAB, such as those supplied by Sasol under the tradename Hyblene^{®}. A suitable anionic detersive surfactant is alkyl benzene sulfonate that is obtained by DETAL catalyzed process, although other synthesis routes, such as HF, may also be suitable. In one aspect a magnesium salt of LAS is used.

Suitable anionic surfactants also include anionic branched surfactants selected from branched sulphate or branched sulphonate surfactants, e.g., branched alkyl sulphate, branched alkyl alkoxylated sulphate, and branched alkyl benzene sulphonates, comprising one or more random alkyl branches, e.g., C1-4 alkyl groups, typically methyl and/or ethyl groups. The branched detersive surfactant may be a mid-chain branched detersive surfactant, e.g., a mid-chain branched anionic detersive surfactant, such as a mid-chain branched alkyl sulphate and/or a mid-chain branched alkyl benzene sulphonate. The branched anionic surfactant may comprise a branched modified alkylbenzene sulfonate (MLAS). The branched anionic surfactant may comprise a C12/13 alcohol-based surfactant comprising a methyl branch randomly distributed along the hydrophobe chain, e.g., Safol^{®}, Marlipal^{®} available from Sasol. Further suitable branched anionic detersive surfactants include those derived from anteiso and iso-alcohols.

Other exemplary anionic surfactants include alkylbenzene sulfonates, alkyl ether sulfates, alkyl sulfates, alkyl and alkenyl ester sulfonates, alkyl and alkenyl sulfonates, isethionates, taurates, or mixture thereof. Any soluble alkali metal or ammonium salt of an anionic surfactant can be used, such as magnesium, sodium, alkanolamine (e.g., triethanolamine), and mixtures thereof. Non-limiting examples of alkylbenzene sulfonates include magnesium alkylbenzene sulfonate, sodium alkylbenzene sulfonate, triethanolamine alkylbenzene sulfonate, alkanolamine alkylbenzene sulfonate, or mixtures thereof. Non-limiting examples of alkyl ether sulfates include magnesium alkyl ether sulfate, sodium alkyl ether sulfate, which may also be referred to as sodium lauryl ether sulfate or sodium laureth sulfate (SLES), triethanolamine alkyl ether sulfate, alkanolamine alkyl ether sulfate, or combinations thereof. Other exemplary anionic surfactants include sodium lauryl sulfate (SLS) and disodium lauryl sulosuccinate.

In some embodiments, the active ingredient composition includes a cationic surfactant. Non-limiting examples of cationic surfactants include: the quaternary ammonium surfactants, which can have up to 26 carbon atoms include: alkoxylate quaternary ammonium (AQA) surfactants, dimethyl hydroxyethyl quaternary ammonium, dimethyl hydroxyethyl lauryl ammonium chloride, polyamine cationic surfactants, cationic ester surfactants, and amino surfactants, e.g., amido propyldimethyl amine (APA).

Suitable cationic detersive surfactants also include alkyl pyridinium compounds, alkyl quaternary ammonium compounds, alkyl quaternary phosphonium compounds, alkyl ternary sulphonium compounds, and mixtures thereof. Other suitable cationic detersive surfactants include quaternary ammonium compounds C8-10 alkyl mono-hydroxyethyl di-methyl quaternary ammonium chloride, mono-C10-12 alkyl mono-hydroxyethyl di-methyl quaternary ammonium chloride and mono-C10 alkyl mono-hydroxyethyl di-methyl quaternary ammonium chloride.

In some embodiments, the active ingredient composition includes an amphoteric surfactant. Examples of amphoteric surfactants include aliphatic derivatives of secondary or tertiary amines, or aliphatic derivatives of heterocyclic secondary and tertiary amines in which the aliphatic radical may be straight or branched-chain and where one of the aliphatic substituents contains at least about 8 carbon atoms, or from about 8 to about 18 carbon atoms, and at least one of the aliphatic substituents contains an anionic water-solubilizing group, e.g. carboxy, sulfonate, sulfate. Examples of compounds falling within this definition are sodium 3-(dodecylamino)propionate, sodium 3-(dodecylamino) propane-1-sulfonate, sodium 2-(dodecylamino)ethyl sulfate, sodium 2-(dimethylamino) octadecanoate, disodium 3-(N-carboxymethyldodecylamino)propane 1-sulfonate, disodium octadecyl-imminodiacetate, sodium 1-carboxymethyl-2-undecylimidazole, and sodium N,N-bis (2-hydroxyethyl)-2-sulfato-3-dodecoxypropylamine. Suitable amphoteric surfactants also include sarcosinates, glycinates, taurinates, and mixtures thereof.

In some embodiments, the active ingredient composition includes a zwitterionic surfactant. Exemplary zwitterionic surfactants include derivatives of secondary and tertiary amines, derivatives of heterocyclic secondary and tertiary amines, or derivatives of quaternary ammonium, quaternary phosphonium or tertiary sulfonium compounds. Suitable examples of zwitterionic surfactants include betaines, including alkyl dimethyl betaine and cocodimethyl amidopropyl betaine, C8 to C18 (for example from C12 to C18) amine oxides and sulfo and hydroxy betaines, such as N-alkyl-N,N-dimethylammino-1-propane sulfonate, where the alkyl group can be C8 to C18.

In some embodiments, the active ingredient composition includes a dispersion aid. Non-limiting examples of dispersion aids include soluble alkali, alkaline earth, and divalent metal salts (e.g., Zn²⁺), polyethylene glycol, poly-glycols, mono or polyhydric alcohols, water-miscible glycols, glycol ethers, polyols, triols, sugar alcohols, glycerin (glycerol), monolaurylglycerol (MLG), polyglycerol esters, hydrotropes, solvents of limited water solubility including without limitation benzyl alcohol, dipropylene glycol n-butyl ether, dipropylene glycol n-propyl ether, phenoxyethanol, or mixtures thereof. In some cases, a cold water dispersion aid can be a soluble magnesium salt. Any suitable water-soluble magnesium salt, including those of organic acids, may be used in the compositions herein. Examples of soluble magnesium salts include, without limitation, magnesium chloride, and magnesium sulfate heptahydrate. Insoluble magnesium ion sources such as magnesium oxide and magnesium hydroxide may be used, provided they are solubilized by reaction with alkylbenzene sulfonic acid or another acidic compound.

In some embodiments, the active ingredient composition includes a starch. Starches can be natural polysaccharides, e.g., derived from plants, composed of linked glucose units. The glucose units can be present in various configurations, including linear starch, e.g., derived from amylose, and branched starches, e.g., derived from amylopectin. In some embodiments, the active ingredient composition includes a modified starch. Exemplary modified starches include acetylated distarch adipate, acetate hexanedioate, hydroxypropyl distarch phosphate, carboxymethyl starch, pregelatinized starches, cationic starches, anionic starches, amphoteric starches, oxidized starches, cross-linked starches, octenyl succinate starch (OSA), waxy starches, e.g., starches with a high amylopectin content, and high amylose starches. Acetate hexanedioate, for example, is a chemically modified starch produced by treating starch with acetic anhydride and hexanedioic acid.

Exemplary cationic starches include hydroxypropyltrimethylammonium chloride (htac), cationic potato starch, cationic corn starch, hydroxypropyl stearoyl ether starch (hm-sta), and cationic rice starch. exemplary anionic starches include acetylated distarch phosphate (adip), phosphated starches, sulfated starches, and acrylamide acrylate copolymer (aa). exemplary amphoteric starches include wheat starch hydrolyzate (wsh), soybean starch, corn starch dialdehyde, cationic/anionic starch, and betaine starch.

Exemplary oxidized starches include sodium hypochlorite oxidized starch, hydrogen peroxide oxidized starch, peracetic acid oxidized starch, dialdehyde starch, and oxidized potato starch. Examples of cationic waxy starches include cationic waxy maize starch, cationic tapioca starch, cationic waxy rice starch, cationic waxy potato starch, and combinations thereof.

In some embodiments, the active ingredient composition includes a preservative. The preservative may include a biocide, antimicrobial, or antifungal compound. In some embodiments, an active ingredient composition includes a non-oxidizing biocide. Exemplary non-oxidizing biocides include glutaraldehyde, 2,2-dibromo-3-nitrilopropionamide (DBNPA), 2-bromo-2-nitropropane-1,3-diol (Bronopol), 5-chloro-2-methyl-4-isothiazolin-3-one (CMIT), isothiazolinones, e.g., 2-methyl-4-isothiazolin-3-one (MIT), a mixture of CMIT and MIT, 1,2-dibromo-2,4-dicyanobutane, bis(trichloromethyl)sulfone, 2-bromo-2-nitrostyrene, 4,5-dichloro-1,2-dithiol-3-one, 2-n-octyl-4-isothiazolin-3-one, 1,2-benzisothiazolin-3-one, ortho-phthalaldehyde, guanidines, biguanidines, pyrithiones, carbamates, 3-iodopropynyl-N-butylcarbamate, phosphonium salts such as tetrakis hydroxymethyl phosphonium sulfate (THPS), 3,5-dimethyl-1,3,5-thiadiazinane-2-thione (Dazomet), 2-(thiocyanomethylthio) benzothiazole, methylene bisthiocyanate (MBT), and combinations thereof.

In some embodiments, an active ingredient composition includes an oxidizing biocide. Examples of oxidizing biocides include chlorine, alkali and alkaline earth hypochlorite salts, hypochlorous acid, chlorinated isocyanurates, bromine, alkali and alkaline earth hypobromite salts, hypobromous acid, bromine chloride, chlorine dioxide, ozone, hydrogen peroxide, peroxy compounds, such as peracetic acid, performic acid, percarbonate or persulfate salts, halogenated hydantoins, e.g., monohalodimethylhydantoins such as monochlorodimethylhydantoin, or dihalodimethylhydantoins such as chlorobromodimethylhydantoin, monochloramines, monobromamines, dihaloamines, trihaloamines, active halogen compounds reacted with other nitrogenous compounds such as urea, and combinations thereof. Other preservatives and preservative systems suitable for use in the present disclosure are described, e.g., in US 2004/0166082 A1 and WO 2022/103729 A1.

Water can also be included in the active ingredient composition provided herein. The amount of water used in the composition can vary depending on the distribution of the other components of the composition. In some cases, some or all of the water can be replaced with one or more of a monohydric alcohol, polyhydric alcohol, water-miscible solvent, or hydrotrope.

In some embodiments, the active ingredient composition includes a fragrance. In some embodiments, the fragrance is natural fragrance. In some embodiments, the fragrance is a synthetic fragrance. In some embodiments, the fragrance comprises an ester. In some embodiments, the fragrance comprises any of allyl amyl glycolate, allyl cyclohexane propionate, amyl acetate, amyl salicylate, benzyl acetate, benzyl salicylate, dodecahydro-3a,6,6,9a-tetramethyl, cis-3-hexenyl acetate, cis-3-hexenyl alicylate, cyclohexyl salicylate, dimethyl benzyl carbinyl acetate, ethyl-2-methyl butyrate, tricyclodecenyl propionate, frutene, geranyl acetate, geranyl nitrile, hexyl acetate, hexyl salicylate, linalyl acetate, resorcyclic acid ester, methyl chavicol, phenyl ethyl acetate, phenyl ethyl phenyl acetate, prenyl acetate, terpinyl acetate, butyl cyclohexyl acetate (verdox), (4-tert-butylcyclohexyl) acetate (vertenex), and combinations thereof. Fragrances available to one of skill in the art may be included, e.g., fragrances that meet the standards of the International Fragrance Association (IFRA) and those described in the art, e.g., WO 2023/232237, WO 2023/232241 A1, US20200048578A1, EP2084254B2, and US20080255024A1.

Other additional components include without limitation, coconut fatty acid, alkylpoly glucoside, cocamidopropyl hydroxysultaine, cocamidopropyl betaine, carbonyl diamide, essential oils, propanediol, isopropylidene glycerol, zinc ricinoleate, tetrasodium glutamate diacetate, ethanol, phenoxyethanol, sodium benzoate, sodium hydroxide, citric acid, sodium citrate, sodium carbonate, sodium carbonate peroxide, sodium chloride, quaternary ammonium compound, calcium chloride, organic acid salt, lactic acid, potassium caprylate, and aloe vera.

In some embodiments, active ingredient compositions include an enzyme or an enzyme blend. Exemplary enzymes include, but are not limited to, hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, B-glucanases, arabinosidases, hyaluronidase, chondroitinase, laccase, amylases, and combinations thereof.

In some embodiments, active ingredient compositions may also include an enzyme stabilizing system. Such stabilizing systems can, for example, comprise calcium ion, boric acid, propylene glycol, short chain carboxylic acids, boronic acids, chlorine bleach scavengers and mixtures thereof, and are designed to address different stabilization problems depending on the type and physical form of the detergent composition. For example, in the case of aqueous detergent compositions comprising protease, a reversible protease inhibitor, such as a boron compound, including borate, 4-formyl phenylboronic acid, phenylboronic acid and derivatives thereof, or compounds such as calcium formate, sodium formate and 1,2-propane diol may be added to further improve stability

In some embodiments, active ingredient compositions include at least one degreasing agent. Non-limiting examples of degreasing agents include, but are not limited to, polyetheramines, polyamines, oligoamines, triamines, diamines, pentamines, tetraamines, tetraethylenepentamine, triethylenetetraamine, diethylenetriamine, or combinations thereof. Specific examples of suitable additional amines include e, or a mixture thereof.

In some embodiments, active ingredient compositions include at least one bleaching agent and/or a bleach catalyst. Suitable bleaching agents other than bleaching catalysts include photobleaches, bleach activators, hydrogen peroxide, sources of hydrogen peroxide, preformed peracids and mixtures thereof.

A bleach catalyst is capable of accepting an oxygen atom from a peroxyacid and/or salt thereof, and transferring the oxygen atom to an oxidizeable substrate. Suitable bleach catalysts include, but are not limited to: iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof.

In some embodiments, active ingredient compositions include at least one brightener. Commercial fluorescent brighteners include derivatives of stilbene, pyrazoline, coumarin, benzoxazoles, carboxylic acid, methinecyanines, dibenzothiophene-5,5-dioxide, azoles, 5- and 6-membered-ring heterocycles, and other miscellaneous agents.

In some embodiments, active ingredient compositions include at least one hueing agent. Typically, the hueing agent provides a blue or violet shade to fabric. Hueing agents include the following known chemical classes of dye: acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof. Hueing agents include dyes, dye-clay conjugates, organic and inorganic pigments, small molecule dyes, and polymeric dyes.

In some embodiments, active ingredient compositions include at least one whitening agent. Whitening agents are capable of eliminating the yellowness exhibited by ageing cellulosic substrates. By utilizing such improved whitening agents, the life of the textile substrates, such as clothing articles, table linens, etc., may be extended. Suitable whitening agents may include dyes, pigments, or polymeric colorants comprising a chromophore constituent and a polymeric constituent. The chromophore constituent is characterized in that it emits or absorbs wavelength in the range of blue, red, violet, purple, or combinations thereof upon exposure to light. Preferably, the chromophore constituent exhibits an absorbance spectrum value from about 520 nanometers to about 640 nanometers in water, and more preferably from about 570 nanometers to about 610 nanometers in water. Preferably, the chromophore constituent exhibits an emission spectrum value from about 400 nanometers to about 480 nanometers in water. Suitable whitening agents include, but are not limited to silicates and carbonates and mixtures thereof.

In some embodiments, active ingredient compositions include at least one dye transfer inhibiting agent. Dye transfer inhibiting agents inhibit the transfer of dyes from one fabric to another during the cleaning process. Generally, such dye transfer inhibiting agents may include polyvinyl pyrrolidone polymers, polyamine N-oxide polymers, copolymers of N-vinylpyrrolidone and N-vinylimidazole, manganese phthalocyanine, peroxidases, and mixtures thereof.

In some embodiments, active ingredient compositions include at least one metal ion chelating agents. Suitable molecules include copper, iron and/or manganese chelating agents and mixtures thereof. Such chelating agents can be selected from the group consisting of phosphonates, amino carboxylates, amino phosphonates, succinates, polyfunctionally-substituted aromatic chelating agents, 2-pyridinol-N-oxide compounds, hydroxamic acids, carboxymethyl inulins and mixtures thereof. Chelating agents can be present in the acid or salt form including alkali metal, ammonium, and substituted ammonium salts thereof, and mixtures thereof. Other suitable chelating agents for use herein are the commercial DEQUEST series, and chelants from Monsanto, Akzo-Nobel, DuPont, Dow, the Trilon^{®} series from BASF and Nalco.

In some embodiments, active ingredient compositions include at least one suds suppressor. Suds suppressors are compounds that reduce or suppress the formation of suds. Examples of suds suppressors include organomodified silicone polymers with aryl or alkylaryl substituents combined with silicone resin and a primary filler, which is modified silicamonocarboxylic fatty acid and soluble salts therein, high molecular weight hydrocarbons such as paraffin, fatty acid esters (e.g., fatty acid triglycerides), fatty acid esters of monovalent alcohols, aliphatic C18-C40 ketones (e.g., stearone), N-alkylated amino triazines, waxy hydrocarbons preferably having a melting point below about 100° C, silicone suds suppressors, and secondary alcohols. Additional suitable antifoams are those derived from phenylpropylmethyl substituted polysiloxanes, e.g., ethylmethyl, methyl(2-phenylpropyl) siloxane .

In some embodiments, active ingredient compositions include at least one suds booster. If high sudsing is desired, suds boosters such as the C10-C16 alkanolamides may be incorporated into disclosed dissolvable sheets or precursor liquid formulations thereof. Some examples include the C10-C14 monoethanol and diethanol amides. If desired, water-soluble magnesium and/or calcium salts to enhance grease removal performance.

In some embodiments, active ingredient compositions include at least one conditioning agent. Suitable conditioning agents include those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein. Suitable conditioning oils include hydrocarbon oils, polyolefins, and fatty esters.

In some embodiments, active ingredient compositions include at least one pH adjuster. The detergent compositions may be formulated such that, during use in aqueous cleaning operations, the wash water will have a pH of between about 7.0 and about 12, and in some examples, between about 7.0 and about 11. Techniques for controlling pH at recommended usage levels include the use of buffers, alkalis, or acids, and are well known to those skilled in the art. These include, but are not limited to, the use of sodium carbonate, citric acid or sodium citrate, lactic acid or lactate, monoethanol amine or other amines, boric acid or borates, and other pH-adjusting compounds well known in the art.

In some embodiments, active ingredient compositions include at least one of a preservative, a carrier, hydrotropes, processing aids, solvents for liquid formulations, and solid or other liquid fillers, erythrosine, colliodal silica, waxes, probiotics, surfactin, aminocellulosic polymers, zinc Ricinoleate, perfume microcapsules, rhamnolipids, sophorolipids, glycopeptides, methyl ester sulfonates, methyl ester ethoxylates, sulfonated estolides, cleavable surfactants, biopolymers, silicones, modified silicones, aminosilicones, deposition aids, locust bean gum, cationic hydroxyethylcellulose polymers, cationic guars, hydrotropes (especially cumenesulfonate salts, toluenesulfonate salts, xylenesulfonate salts, and naphalene salts), antioxidants, BHT, pearlescent agents, effervescent agents, color change systems, silicone polyurethanes, opacifiers, tablet disintegrants, biomass fillers, fast-dry silicones, glycol distearate, hydroxyethylcellulose polymers, hydrophobically modified cellulose polymers or hydroxyethylcellulose polymers, starch perfume encapsulates, emulsified oils, bisphenol antioxidants, microfibrous cellulose structurants, properfumes, styrene/acrylate polymers, triazines, soaps, superoxide dismutase, benzophenone protease inhibitors, functionalized TiO2, dibutyl phosphate, silica perfume capsules, and other adjunct ingredients, silicate salts (e.g., sodium silicate, potassium silicate), choline oxidase, pectate lyase, mica, titanium dioxide coated mica, bismuth oxychloride, xanthan gum, cellulose gum, and sodium benzoate, and other actives.

A laundry sheet or laundry detergent sheet is one example of a preferred embodiment of disclosed dissolvable matrix compositions. The laundry sheet comprises water in an amount as low as 1 wt.%. 2 wt.%. 3 wt.%. 4 wt.%. 5 wt.%. 6 wt.%, 7 wt.%, 8 wt.%, 9 wt.%, 10 wt.%, or as high as 11 wt.%, 12 wt.%, 13 wt.%, 14 wt.%, 15 wt.%, 16 wt.%, 17 wt.%, 18 wt.%, 19 wt.%, 20 wt.%, or within any range encompassed by any two of the foregoing values as endpoints. For example, the water may be present in an amount of from 1 wt.%, to 8 wt.%, from 2 wt.%, to 7 wt.%, or from 3 wt.%, to 5 wt.%.

The laundry sheet includes a nonionic surfactant. The nonionic surfactant may include at least one C₁₀ to C₁₆ ethoxylated alcohol. Relative to the total weight of the sheet composition, the nonionic surfactant may be present in an amount as low as 10 wt.%, 11 wt.%, 12 wt.%, 13 wt.%, 14 wt.%, 15 wt.%, 16 wt.%, 17 wt.%, 18 wt.%, 19 wt.%, or 20 wt.%, or as high as 21 wt.%, 22 wt.%, 23 wt.%, 24 wt.%, 25 wt.%, 26 wt.%, 27 wt.%, 28 wt.%, 29 wt.%, 30 wt.%, 31 wt.%, 32 wt.%, 33 wt.%, 34 wt.%, 35 wt.%, 36 wt.%, 37 wt.%, 38 wt.%, 39 wt.%, 40 wt.%, 41 wt.%, 42 wt.%, 43 wt.%, 44 wt.%, 45 wt.%, or within any range encompassed by any two of the foregoing values as endpoints. For example, at least one C₁₀ to C₁₆ ethoxylated alcohol may be present in an amount of from 10 wt.% to 40 wt.%, from 31 wt.% to 39 wt.%, from 32 wt.% to 38 wt.%, 33 wt.% to 37 wt.%, or 34 wt.% to 36 wt.% relative to the total weight of the laundry sheet composition.

The laundry sheet includes an anionic surfactant. The anionic surfactant may include sodium and ammonium alkyl and alkyl ether sulfates, e.g., SLS or SLES. The sheet may include the anionic surfactant in an amount as low as 1 wt.%. 2 wt.%. 3 wt.%. 4 wt.%. 5 wt.%. 6 wt.%, 7 wt.%, 8 wt.%, 9 wt.%, 10 wt.%, or within any range encompassed by any two of the foregoing values as endpoints. For example, the sodium lauryl ether sulfate may be present in an amount of from 1 wt.% to 10 wt.%, from 6 wt.% to 10 wt.%, or from 7 wt.% to 9 wt.% relative to the total weight of the laundry sheet composition.

In some examples, the laundry sheet includes an enzyme blend. The enzyme blend may include any one of a protease enzyme, an amylase enzyme, a pectate lyase enzyme, a mannanase enzyme, a lipase enzyme, or a combination thereof. Relative to the total weight of the sheet composition, the sheet may include the enzyme blend in a total amount as low as 1 wt.%. 2 wt.%. 3 wt.%. 4 wt.%. 5 wt.%. 6 wt.%, 7 wt.%, 8 wt.%, or within any range encompassed by any two of the foregoing values as endpoints. For example, the sheet may comprise the enzyme blend in an amount of from 2 wt.% to 4 wt.%.

The laundry sheet includes a starch. The starch may include a modified starch, e.g., an acetylated starch. Relative to the total weight of the sheet composition, the starch can be present in an amount as low as 1 wt.%. 2 wt.%. 3 wt.%. 4 wt.%. 5 wt.%. 6 wt.%, 7 wt.%, 8 wt.%, or within any range encompassed by any two of the foregoing values as endpoints. For example, the sheet may comprise acetate hexanedioate starch in an amount of from 1 wt.% to 5 wt.% or from 2 wt.% to 4 wt.% relative to the total weight of the sheet composition.

In some examples, the laundry sheet includes a dispersion aid. The dispersion aid can include glycerin. Relative to the total weight of the sheet composition, the sheet may include the dispersion aid in a total amount as low as 1 wt.%. 2 wt.%. 3 wt.%. 4 wt.%. 5 wt.%. 6 wt.%, 7 wt.%, 8 wt.%, 9 wt.%, or 10 wt.%, or as high as 11 wt.%, 12 wt.%, 13 wt.%, 14 wt.%, 15 wt.%, 16 wt.%, 17 wt.%, 18 wt.%, 19 wt.%, or 20 wt.%, or within any range encompassed by any two of the foregoing values as endpoints. For example, the sheet may comprise glycerin in an amount of from 1 wt.% to 10 wt.% or from 2 wt.% to 4 wt.%.

The laundry sheet includes 0.01 wt.% to 0.5 wt.% of a preservative. In some examples, the laundry sheet includes a biocide and a preservative. The biocide and/or the preservative may include an isothiazolinone, e.g., 2-methyl-4-thiazoline-3-ketone. Relative to the total weight of the laundry sheet composition, a biocide and/or a preservative can be present in a total amount as low as 0.01 wt.%, 0.02 wt.%, 0.03 wt.%, 0.04 wt.%, 0.05 wt.%, 0.06 wt.%, 0.07 wt.%, 0.08 wt.%, 0.09 wt.%, 0.10 wt.%, 0.11 wt.%, 0.12 wt.%, 0.13 wt.%, 0.14 wt.%, 0.15 wt.%, 0.16 wt.%, 0.17 wt.%, 0.18 wt.%, 0.19 wt.%, 0.20 wt.%, 0.21 wt.%, 0.22 wt.%, 0.23 wt.%, or as high as 0.24 wt.%, 0.25 wt.%, 0.26 wt.%, 0.27 wt.%, 0.28 wt.%, 0.29 wt.%, 0.30 wt.%, 0.31 wt.%, 0.32 wt.%, 0.33 wt.%, 0.34 wt.%, 0.35 wt.%, 0.36 wt.%, 0.37 wt.%, 0.38 wt.%, 0.39 wt.%, 0.40 wt.%, or within any range encompassed by any two of the foregoing values and endpoints. For example, the 2-methyl-4-thiazoline-3-ketone may be present in an amount of from 0.10 wt.% to 0.40 wt.%, from 0.12 wt.% to 0.30 wt.%, from 0.15 wt.% to 0.25 wt.%, or from 0.18 wt.% to 0.23 wt.%.

Relative to the total weight of the sheet composition, the sheet may include a fragrance in an amount as low as 1 wt.%, 2 wt.%, 3 wt.%, 4 wt.%, 5 wt.%, 6 wt.%, 7 wt.%, 8 wt.%, 9 wt.%, 10 wt.%, or as high as 11 wt.%, 12 wt.%, 13 wt.%, 14 wt.%, 15 wt.%, 16 wt.%, 17 wt.%, 18 wt.%, 19 wt.%, 20 wt.%, or within any range encompassed by any two of the foregoing values as endpoints. For example, the sheet may comprise a fragrance in an amount of from 1 wt.% to 6 wt.%, from 2 wt.% to 5 wt.%, or from 3 wt.% to 4 wt.%.

### ii. Personal Care Active Ingredients

In some embodiments, disclosed dissolvable sheets contain an active ingredient composition that includes at least one personal care active ingredient. Personal care active ingredients, also referred to as personal hygiene active ingredients, include but are not limited to shampoo, conditioner, body wash, facial cleanser, moisturizers, makeup remover, deodorant, sanitizers, antibacterials, sunscreen, and ingredients for pet grooming. Personal care ingredients often include surfactants, such as the anionic, nonionic, cationic, zwitterionic or amphoteric surfactants described herein.

More specifically, active ingredient compositions can include personal care active ingredients such as but not limited to lipids, hydrocarbons, fats, oils, hydrophobic plant extracts, fatty acids, essential oils, silicone materials and mixtures thereof, vitamins and derivatives thereof, sunscreens, preservatives, anti-acne medicaments, antioxidants, skin soothing and healing, chelators and sequestrants, essential oils, and mixtures thereof.

In some embodiments, active ingredient compositions include a lipophilic material. Lipophilic material as used herein means a lipophilic active typically delivered to an external surface of the human body to enhance or improve a characteristic of the surface. The lipophilic material includes any water insoluble, oil miscible, or lipophilic substance which benefits the keratinous substrate. The active ingredient composition may include one lipophilic material or a mixture of two or more lipophilic materials. Non-limiting examples of lipophilic materials suitable for use according to the present disclosure include essential oils, fragrance oils, ester oils, glyceride oils, plant oils, fatty alcohols, silicone oils, hydrocarbon oils, petrolatum, plant waxes, plant butters, water insoluble or slightly soluble organic sunscreens, micronized sunscreens, plant extracts, hydrophobically modified pigments (e.g., Natural Orange 4 (CI75120), D&C Violet 2 (CI60725), D&C Red 17 (CI26100), D&C Green 6 (CI61565), D&C Red 6 or Red 7(CI15850), and Quinoline Yellow SS (47000)), hydrophobic vitamin or vitamin complexes, antioxidants, antifungal agents (e.g., zinc pyrithione), anti-inflammatory actives, antimicrobials, antiperspirant actives, deodorant actives, skin health actives, and mixtures thereof.

Additional examples of lipophilic materials include a variety of hydrocarbons, oils and waxes, silicones, fatty acid derivatives, cholesterol, cholesterol derivatives, diglycerides, triglycerides, vegetable oils, vegetable oil derivatives, acetoglyceride esters, alkyl esters, alkenyl esters, polyglycerin fatty acid esters, lanolin and its derivatives, wax esters, beeswax derivatives, sterols and phospholipids, vitamins and pro-vitamins and combinations thereof. Non-limiting examples of hydrocarbon oils and waxes suitable for use herein include petrolatum, mineral oil, micro-crystalline waxes, polyalkenes, paraffins, cerasin, ozokerite, polyethylene, perhydrosqualene, and combinations thereof. Non-limiting examples of silicone oils suitable for use as hydrophobic benefit components herein include dimethicone copolyol, dimethylpolysiloxane, diethylpolysiloxane, mixed C1-C30 alkyl polysiloxanes, phenyl dimethicone, dimethiconol, and combinations thereof. Preferred are non-volatile silicones selected from dimethicone, dimethiconol, mixed C1-C30 alkyl polysiloxane, and combinations thereof. Nonlimiting examples of silicone oils useful herein are described in U.S. Patent No. 5,011,681.

Exemplary essential oils include, but are not limited to, oil of eucalyptus, oil of hybrid lavender, oil of lavender, oil of vetiver, oil of Litsea cubeba, oil of lemon and lime (limonene), cinnamaldehyde, oil of cinnamon (bark), oil of cinnamon (leaf), oil of sandalwood, oil of rosemary, oil of chamomile, oil of savory, oil of nutmeg, oil of cinnamon, oil of hyssop, oil of caraway, oil of orange, oil of tea tree, oil of geraniol, oil of eugenol, oil of carvacrol, oil of prickly juniper, and oil of bergamot.

Fragrance oils, unlike the naturally derived essential oils, are synthetically manufactured scents. Exemplary fragrance oils are anethol, methyl heptine carbonate, ethyl aceto acetate, para cymene, nerol, decyl aldehyde, para cresol, methyl phenyl carbinyl acetate, ionone alpha, ionone beta, undecylenic aldehyde, undecyl aldehyde, 2,6-nonadienal, nonyl aldehyde, octyl aldehyde, phenyl acetaldehyde, anisic aldehyde, benzyl acetone, ethyl-2-methyl butyrate, damascenone, damascone alpha, damascone beta, for acetate, frutene, fructone, herbavert, methyl isobutenyl tetrahydropyran, isopropyl quinoline, 2,6-nonadien-1-ol, 2-methoxy-3-(2-methylpropyl)-pyrazine, methyl octine carbonate, cyclogalbanate, gamma nonalactone, cis 1,3-oxathiane-2-methyl-4-propyl, benzaldehyde, benzyl acetate, camphor, carvone, borneol, bornyl acetate, decyl alcohol, eucalyptol, linalool, hexyl acetate, iso-amyl acetate, thymol, carvacrol, limonene, menthol, iso-amyl alcohol, phenyl ethyl alcohol, alpha pinene, alpha terpineol, citronellol, alpha thujone, benzyl alcohol, beta gamma hexenol, dimethyl benzyl carbinol, phenyl ethyl dimethyl carbinol, adoxal, allyl cyclohexane propionate, beta pinene, citral, citronellyl acetate, citronellal nitrile, dihydro myrcenol, geraniol, geranyl acetate, geranyl nitrile, hydroquinone dimethyl ether, hydroxycitronellal, linalyl acetate, phenyl acetaidehyde dimethyl acetal, phenyl propyl alcohol, prenyl acetate, tetrahydrolinalool, verdox, and cis-3-hexenyl acetate, ethyl methyl phenyl glycidate, ethyl vanillin, heliotropin, Indol, methyl anthranilate, vanillin, amyl salicylate, coumarin, ambrox, bacdanol, benzyl salicylate, butyl anthranilate, cetalox, ebanol, cis-3-hexenyl salicylate, lilial, gamma undecalactone, gamma dodecalactone, gamma decalactone, calone, cymal, dihydro iso jasmonate, iso eugenol, lyral, methyl beta naphthyl ketone, beta naphthol methyl ether, para hydroxy I phenyl butanone, 8-cyclohexadecen-1-one, oxocyclohexadecen-2-one/habanolide, florhydral, intreleven aldehyde eugenol, amyl cinnamic aldehyde, hexyl cinnamic aldehyde, hexyl salicylate, methyl dihydro jasmonate, sandalore, veloutone, undecavertol, exaltolide/cyclopentadecanolide, zingerone, methyl cedrylone, sandela, dimethyl benzyl carbinyl butyrate, dimethyl benzyl carbinyl isobutyrate, triethyl citrate, cashmeran, phenoxy ethyl isobutyrate, iso eugenol acetate, helional, Iso E Super^{™}, ionone gamma methyl, pentalide, galaxolide, phenoxy ethyl propionate, and mixtures thereof.

The ester oils are characterized by having at least 12 carbon atoms and include esters with hydrocarbon chains derived from fatty acids or alcohols, e.g., mono-esters, polyhydric alcohol esters, and di-and tri-carboxylic acid esters. The hydrocarbyl radicals of the fatty esters hereof can also include or have covalently bonded thereto other compatible functionalities, such as amides and alkoxy moieties (e.g., ethoxy or ether linkages).

The monocarboxylic acid esters include esters of alcohols and/or acids of the formula R'COOR wherein the alkyl or alkenyl radicals and the sum of carbon atoms in R' and R is at least 10, or at least 20.

Ester oils include, for example, alkyl and alkenyl esters of fatty acids having aliphatic chains having from about 10 to about 22 carbon atoms, and alkyl and alkenyl fatty alcohol carboxylic acid esters having an alkyl and/or alkenyl alcohol derived aliphatic chain with about 10 to about 22 carbon atoms, and combinations thereof. Examples include lauryl lactate, myristyl lactate, cetyl lactate, hexyl laurate, isohexyl laurate, myristyl myristate, cetyl myristate, stearyl myristate, isostearyl myristate, oleyl myristate, behenyl myristate, erucyl myristate, isopropyl palmitate, isohexyl palmitate, myristyl palmitate, cetyl palmitate, stearyl palmitate, isostearyl palmitate, oleyl palmitate, behenyl palmitate, erucyl palmitate, isopropyl isostearate, decyl stearate, myristyl stearate, myristyl isostearate, cetyl stearate, cetyl isostearate, stearyl stearate, stearyl isostearate, isostearyl stearate, isostearyl isostearate, oleyl stearate, oleyl isostearate, behenyl stearate, behenyl isostearate, erucyl stearate, erucyl isostearate, decyl oleate, isodecyl oleate, myristyl oleate, cetyl oleate, stearyl oleate, isostearyl oleate, oleyl oleate, behenyl oleate, erucyl oleate, myristyl behenate, cetyl behenate, stearyl behenate, isostearyl behenate, oleyl behenate, behenyl behenate, erucyl behenate, myristyl erucate, cetyl erucate, oleyl erucate, and erucyl erucate.

Di- and tri-alkyl and alkenyl esters of carboxylic acids can also be used. These include, for example, esters of C4-C8 dicarboxylic acids such as C1-C22 esters of succinic acid, glutaric acid, adipic acid, hexanoic acid, heptanoic acid, and octanoic acid. Representative examples include isocetyl stearyl stearate, diisopropyl adipate, diisohexyl adipate, dihexyldecyl adipate, diisopropyl sebacate and tristearyl citrate. Polyhydric alcohol esters include alkylene glycol esters (di-fatty acid esters), ethylene glycol (mono- and di-fatty acid esters), polyethylene glycol (mono and di-fatty acid esters), propylene glycol (mono- and di-fatty acid esters), polypropylene glycol mono oleate, polypropylene glycol 2000 monostearate, ethoxylated propylene glycol monostearate, glyceryl mono-and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, polyoxyethylene sorbitan fatty acid esters are satisfactory polyhydric alcohol esters for use as ester oils.

Representative glyceride oils include, but are not limited to, di- and tri-esters of glycerol and fatty acids having 6 to 22, or 8 to 18, or 10 to 16 carbon atoms, and mixtures thereof. In one aspect, the fatty acids utilized to prepare the glyceride oils can be saturated or unsaturated, linear or branched, substituted or unsubstituted. Exemplary glyceride oils include, but are not limited to, acetoglyceryl, glyceryl triisooctanoate, glyceryl triisostearate, glyceryl triisopalmitate, glyceryl tri(caprylate.caprate), glyceryl monostearate, glyceryl di-2-heptylundecanoate, glyceryl trimyristate, diglyceryl myristate isostearate, caprylic/capric triglyceride, and mixtures thereof.

Exemplary plant oils include olive oil, sunflower oil, soya oil, groundnut oil, peanut oil, rapeseed oil, sweet almond oil, jojoba oil, borage oil, palm oil, coconut oil, castor oil, hydrogenated castor oil, barley oil, walnut oil, wheatgerm oil, grapeseed oil, evening primrose oil, macadamia nut oil, babassu oil, carrot oil, palm kernel oil, shea butter oil, sesame oil, peach stone oil, corn oil, karite butter, apricot oil, cottonseed oil, alfalfa oil, poppy oil, pumpkin oil, marrow oil, grapefruit seed oil, avocado oil, hazelnut oil, blackcurrant seed oil, millet oil, barley oil, rye oil, quinoa oil, olive oil, rye oil, safflower oil, candlenut oil, passiflora oil, passion flower oil, musk rose oil, camellia oil, camelina oil, pine oil, tamanu oil, and mixtures thereof.

Exemplary fatty alcohols are selected from linear and branched, saturated and unsaturated C12 to C30 fatty alcohols. Non-limiting examples of fatty alcohols are lauryl alcohol, myristyl alcohol, cetyl alcohol, isocetyl alcohol, stearyl alcohol, isostearyl alcohol, cetearyl alcohol, palmitoleyl alcohol, elaidyl alcohol, sterol, oleyl alcohol, linoleyl alcohol, elaidolinoleyl alcohol, linolenyl alcohol, ricinoleyl alcohol, arachidyl alcohol, icocenyl alcohol, behenyl alcohol, erucyl alcohol, lignoceryl alcohol, ceryl alcohol, montanyl alcohol, myricyl alcohol, tridecyl alcohol, and mixtures thereof. Fatty alcohols are widely available and can be obtained through the hydrogenation of esterified vegetable and animal oils and fats.

Silicone oils are synthetic polymeric compounds in which the silicon atoms are bonded together via oxygen atoms. In one aspect, the silicone oil is non-volatile and insoluble in the aqueous phase of the present composition. By non-volatile is meant that the silicone has a very low vapor pressure at ambient temperature conditions (e.g., less than 2 mm Hg at 20° C.). The non-volatile silicone conditioning agent has a boiling point above about 250° C., or above about 260° C., or above about 275° C. in a further aspect. Background information on silicones including sections discussing silicone oils, gums, and resins, as well as their manufacture, are found in Encyclopedia of Polymer Science and Engineering, vol. 15, 2d ed., pp 204-308, John Wiley & Sons, Inc. (1989).

Suitable silicone oils include, but are not limited to, higher alkoxy-modified silicones and higher fatty acid-modified silicones such as dimethylpolysiloxane, methylphenylpolysiloxane, methylhydrogenpolysiloxane, octamethylcyclotetrasiloxane, decamethylcyclopentasiloxane, dodecamethylcyclohexasiloxane, tetramethyltetrahydrogencyclotetrasiloxane, and stearoxysilicone as well as fluorine-modified silicones, amino-modified silicones, alkyl-modified silicones, and mixtures thereof.

Hydrocarbon oils include volatile hydrocarbon oils, non-volatile hydrocarbon oils, and mixtures thereof. Suitable volatile hydrocarbon oils include linear or branched, optionally cyclic, C5-C20 lower alkanes. Examples include, but are not limited to pentane, hexane, heptane, decane, undecane, dodecane, tridecane, tetradecane and C8-C18 isoparaffins, for example, isodecane, isododecane and isohexadecane.

The hydrocarbon oils may be paraffinic hydrocarbons including C12 and C16 isoparaffins. Such isoparaffinic hydrocarbons are available from ExxonMobil under the Isopars^{™} trade name, and from the Permethyl Corporation marketed under the Permethyl^{™} trade name. Exemplary C12 isoparaffins (isododecane) are commercially available from Permethyl Corporation under the trade name Permethyl 99A. A C16 isoparaffin (isohexadecane) that is commercially available under the Permethyl 101A tradename, is also suitable. Other examples of hydrocarbon oils include polydecene, polyisobutene, petrolatum and mineral oil. Mixtures of hydrocarbon oils are contemplated.

Plant waxes include, but are not limited to, olive tree wax, rice wax, carnauba wax, fruit waxes, hydrogenated jojoba wax or the absolute waxes of flowers such as the essential wax of blackcurrant flower marketed by Huiles Bertin, lanolin, beeswaxes, modified beeswaxes, marine waxes, and mixtures thereof.

Plant butters are butter-like compositions made with plant-based oils. Such butters are generally characterized by their hydrocarbon chain length as taught in U.S. Pat. No. 5,554,408. Non-limiting plant butters useful in the present compositions include, almond butter, hemp seed butter, shea butter, cocoa butter, coconut butter, aloe butter, jojoba butter, lavender butter, lemon butter, lime butter orange peel butter, tangerine butter, soy butter, tucuma butter, turmeric butter, ucuuba butter, rose hip butter, argan butter, arnica butter, macadamia butter, avocado butter, mango butter, bacuri butter, chamomile butter, chia butter, carrot butter, green tea butter, olive butter, moringa butter, oat butter, prickly pear butter, pumpkin seed butter, kokum butter, murumuru butter, cupuacu butter, babassu butter, and mixtures thereof. Mixtures of one or more of the foregoing oily materials are contemplated within the scope of the disclosure. Additional lipophilic materials or hydrophobic compounds suitable for inclusion include those described in WO2005084615A1.

In some embodiments, active ingredient compositions include humectants. A preferred humectant is glycerin. In some embodiments, active ingredient compositions include a water soluble, organic material. A preferred water soluble, organic material is selected from the group consisting of a polyol of the structure: Rl - O(CH2 - CR2HO)nH where Rl = H, C1-C4 alkyl; R2 = H, CH3 and n = 1 - 200; C2-C10 alkane diols; guanidine; glycolic acid and glycolate salts (e.g. ammonium and quaternary alkyl ammonium); lactic acid and lactate salts (e.g. ammonium and quaternary alkyl ammonium); polyhydroxy alcohols such as sorbitol, glycerol, hexanetriol, propylene glycol, hexylene glycol and the like; polyethylene glycol; sugars and starches; sugar and starch derivatives (e.g. alkoxylated glucose); panthenol (including D-, L-, and the Deforms); pyrrolidone carboxylic acid; hyaluronic acid; lactamide monoethanolamine; acetamide monoethanolamine; urea; and mixtures thereof. The most preferred polyols are selected from the group consisting of glycerine, polyoxypropylene(l) glycerol and polyoxypropylene glycerol, sorbitol, butylene glycol, propylene glycol, sucrose, urea and triethanol amine. Nonionic polyethylene/polypropylene glycol polymers are preferably used as skin conditioning agents.

Polymers useful herein that are especially preferred are PEG-2M wherein x equals 2 and n has an average value of about 2,000 (PEG 2-M is also known as Polyox WSR^{®} N-10 from Union Carbide and as PEG-2,000); PEG-5M wherein x equals 2 and n has an average value of about 5,000 (PEG 5-M is also known as Polyox WSR^{®} 35 and Polyox WSR^{®} N-80, both from Union Carbide and as PEG-5,000 and Polyethylene Glycol 200,000); PEG-7M wherein x equals 2 and n has an average value of about 7,000 (PEG 7-M is also known as Polyox WSR^{®} (N-750 from Union Carbide); PEG-9M wherein x equals 2 and n has an average value of about 9,000 (PEG 9-M is also known as Polyox WSR^{®} N-3333 from Union Carbide); PEG-14 M wherein x equals 2 and n has an average value of about 14,000 (PEG 14-M is also known as Polyox WSR- 205 and Polyox WSR^{®} N-3000 both from Union Carbide); and PEG-90M wherein x equals 2 and n has an average value of about 90,000. (PEG-90M is also known as Polyox WSR^{®}-301 from Union Carbide.)

In some embodiments, active ingredient compositions include vitamins and derivatives thereof (e.g., ascorbic acid, vitamin E, tocopheryl acetate, and the like); sunscreens; thickening agents (e.g., polyol alkoxy ester, available as Crothix from Croda); preservatives for maintaining the anti-microbial integrity of the cleansing compositions; anti-acne medicaments (resorcinol, salicylic acid, and the like); antioxidants; skin soothing and healing agents such as aloe vera extract, allantoin and the like; chelators and sequestrants; and agents suitable for aesthetic purposes such as fragrances, essential oils, skin sensates, pigments, pearlescent agents (e.g., mica and titanium dioxide), lakes, colorings, and the like (e.g., clove oil, menthol, camphor, eucalyptus oil, and eugenol). Still other suitable hydrophobic benefit components include ethanol amines of the general structure (HOCH2CH2)xNHy where x = 1-3; y = 0-2, and x+y = 3.

In some embodiments, an active ingredient composition contains a particle. Preferably, the particle will also have physical properties which are not significantly affected by typical processing of the composition. For example, a particle having a melting point greater than about 50°C, 60°C, or 70°C is used. As used herein, melting point would refer to the temperature at which the particle transitions to a liquid or fluid state or undergoes significant deformation or physical property changes. Particles can be cross-linked or have a cross-linked surface membrane, which do not exhibit a distinct melting point. Cross-linked particles are also useful as long as they are stable under the processing and storage conditions used in the making of compositions.

The particles that can be present in the present invention can be natural, synthetic, or semi-synthetic. In addition, hybrid particles can also be present. Synthetic particles can made of either cross-linked or non-cross-linked polymers. The particles of the present invention can have surface charges or their surface can be modified with organic or inorganic materials such as surfactants, polymers, and inorganic materials. Particle complexes can be present. Non limiting examples of natural particles include various precipitated silica particles in hydrophilic and hydrophobic forms available from Degussa-Huls under the trade name Sipernet. Precipitated^{™}, hydrophobic, synthetic amorphous silica, available from Degussa under the trade name Sipernet Dll^{™} is a preferred particle. Snowtex colloidal silica particles available from Nissan Chemical America Corporation. Nonlimiting examples of synthetic particles include nylon, silicone resins, poly(meth)acrylates, polyethylene, polyester, polypropylene, polystyrene, polyurethane, polyamide, epoxy resins, urea resins, and acrylic powders. Non limiting examples of useful particles are Microease 11 OS, 114S, 116 (micronized synthetic waxes), Micropoly 210, 250S (micronized polyethylene), Microslip (micronized polytetrafluoroethylene), and Microsilk (combination of polyethylene and polytetrafluoroethylene), all of which are available from Micro Powder, Inc. Additional examples include Luna (smooth silica particles) particles available from Phenomenex, MP-2200 (polymethylmethacrylate), EA- 209 (ethylene/acrylate copolymer), SP-501(nylon-12), ES-830 (polymethly methacrylate), BPD-800, BPD-500 (polyurethane) particles available from Kobo Products, Inc. and silicone resins sold under the name Tospearl particles by GE Silicones. Ganzpearl GS-0605 crosslinked polystyrene (available from Presperse) is also useful. Non limiting examples of hybrid particles include Ganzpearl GSC-30SR (Sericite & crosslinked polystyrene hybrid powder), and SM-1000, SM-200 (mica and silica hybrid powder available from Presperse).

In some examples the particle is an exfoliating particle. The exfoliant particle is selected from the group consisting of polyethylene, microcryatalline wax, jojoba esters, amourphors silica, talc, tracalcium orthophosphate, or blends thereof, and the like. The exfoliant particle has a particle size dimension along the major axis of the particle of from about 100 microns to about 600 microns, preferably from about 100 microns to about 300 microns. The exfoliant particle has a hardness of less than about 4 Mohs, preferably less than about 3 Mohs. The hardness as so measured is a criterion of the resistance of a particular material to crushing. It is known as being a fairly good indication of the abrasive character of a particulate ingredient. Examples of materials arranged in increasing order of hardness according to the Moh scale are as follows: h(hardness)-l:talc; h-2: gypsum, rock salt, crystalline salt in general, barytes, chalk, brimstone; h-4: fluorite, soft phosphate, magnesite, limestone; h-5: apatite, hard phosphate, hard limestone, chromite, bauxite; h-6: feldspar, ilmenite, hornblendes; h-7: quartz, granite; h-8: topaz; h-9: corrundum, emery; and h-10: diamond.

In some embodiments, the active ingredient composition includes hair care ingredients, e.g., shampoo or conditioner. Shampoo or conditioner compositions may comprise a combination of surfactants and conditioning agents. Accordingly, active ingredient compositions can include one or more surfactants as well as conditioning agents such as silicones, hydrocarbon oils, fatty esters or combinations thereof. In shampoo compositions containing conditioning agents, deposition of the conditioning agent can be improved by including certain cationic deposition polymers. These cationic deposition polymers may be natural polymers such as cellulose polymers or guar polymers that have been modified with cationic substituents.

Active ingredient compositions including shampoo or conditioner formulations may include one or more surfactants. Surfactants are included to provide cleaning performance to the composition and may include an amphoteric surfactant, a zwitterionic surfactant, a cationic surfactant, a nonionic surfactant, and optionally at least one other surfactant, which may be an amphoteric surfactant, a zwitterionic surfactant, a cationic surfactant, a nonionic surfactant, or a combination thereof. Such surfactants should be physically and chemically compatible with the essential components described herein, or should not otherwise unduly impair product stability, aesthetics or performance.

Active ingredient compositions including shampoo or conditioner formulations may include an oily conditioning agent. Such conditioning agents include materials used to impart specific conditioning benefits to the hair and/or skin. In hair treatment compositions, suitable conditioning agents are those which provide one or more benefits related to shine, softness, combability, antistatic properties, wet-handling, damage, manageability, body and greasiness. Oil conditioning agents useful in shampoo formulations of the present disclosure may comprise a water insoluble, water dispersible, nonvolatile liquid that forms emulsified liquid particles. Suitable oily conditioning agents are those typically characterized as silicones (e.g., silicone oils, cationic silicones, silicone rubbers, high refractive silicones, and silicone resins), organic conditioning oils (e.g., hydrocarbon oils, polyolefins, and fatty esters), or combinations thereof, or those that otherwise form liquid dispersed particles in the aqueous surfactant matrix herein.

Other conditioning agents, such as quaternary ammonium compounds, may also be included in the shampoo formulation. Quaternary ammonium compounds suitable for use as conditioning agents in the personal care compositions of the present invention include, but are not limited to, hydrophilic quaternary ammonium compounds having a long chain substituent containing a carbonyl moiety (e.g., an amide moiety) or a phosphate moiety or similar hydrophilic moiety.

Examples of useful hydrophilic quaternary ammonium compounds include, but are not limited to, compounds designated as ricinoleic acid amidopropyl trimethyl ammonium chloride (ricinoleic acid amidopropyl trimethyl ammonium chloride), ricinoleic acid amidotrimethyl ammonium ethyl sulfate (ricinoleic acid amidopropyl trimethyl ammonium ethyl sulfate), hydroxystearamidopropyl trimethyl ammonium methyl sulfate (hydroxystearamidopropyl trimethyl ammonium methyl sulfate), and hydroxystearamidopropyl trimethyl ammonium chloride (hydroxystearamidopropyl trimethyl ammonium chloride), in the CTFA Cosmetic Dictionary (CTFA Cosmetic Dictionary), or combinations thereof.

Other examples of useful quaternary ammonium surfactants include, but are not limited to, quaternium-16, quaternium-27, quaternium-30, quaternium-52, quaternium-53, quaternium-56, quaternium-60, quaternium-61, quaternium-62, quaternium-63, quaternium-71, quaternium-33, quaternium-43, isostearamidopropylethyldimethylammonium ethyl sulfate, quaternium-22, and Quaternium-26, or combinations thereof, as specified in the CTFA dictionary.

Active ingredient compositions may also include a water-soluble cationically modified starch polymer. The term "cationically modified starch" as used herein refers to starches to which cationic groups have been added before the starch has degraded to a smaller molecular weight or starches to which cationic groups have been added after the starch has been modified to a desired molecular weight. The term "cationically modified starch" is also defined to include amphiphilically modified starches. The term "amphiphilically modified starch" refers to a starch hydrolysate to which cationic and anionic groups have been added.

Active ingredient compositions may include cationically modified starch polymers, e.g., those suitable for shampoo or conditioner formulations, such as maltodextrin. Thus, in one example, the cationically-modified starch polymer can be polymerized with less than about 35, and more preferably from about 1 or more to about 20 or less, grape sugar equivalent ("DE") values for further characterization. The DE value is a measure of the reducing equivalents of hydrolyzed starch relative to glucose and is expressed as a percentage (dry weight basis). Starch that is completely hydrolyzed to glucose has a DE value of 100 and unhydrolyzed starch has a DE value of 0. Suitable determinations of DE values include "Dextrose Equisitive",Standard Analytical Methods of the Member Companies of the Corn Industries Research Foundation1 st edition, method E-26. Furthermore, the cationically modified starch polymer of the invention may comprise dextrin. Dextrins are generally pyrolysis products of starch having a wide range of molecular weights.

The source of starch before chemical modification may be selected from a variety of sources such as tubers, legumes, cereals and grains. Non-limiting examples of such starch sources may include corn starch, wheat starch, rice starch, waxy corn starch, oat starch, tapioca starch, waxy barley, waxy rice (waxy rice) starch, waxy rice (glutenous rice) starch, waxy rice (sweet rice) starch, amylopectin (amioca) starch, potato starch, tapioca starch, oat starch, sago starch, waxy rice (sweet rice) starch, or mixtures thereof.

In one example, the cationically modified starch polymer is selected from the group consisting of degraded cationic corn starch, cationic tapioca, cationic potato starch and mixtures thereof. In another example, the cationically modified starch polymer is cationic corn starch. The starch may comprise one or more additional modifications before degradation or after modification to a lower molecular weight. For example, these modifications may include crosslinking, stabilization reactions, phosphorylation, and hydrolysis. The stabilization reaction may include alkylation and esterification.

The active ingredient composition may include cationically modified starch polymers in the form of hydrolyzed starch (e.g., acid, enzymatic, or alkaline degradation), oxidized starch (e.g., peroxide, peracid, hypochlorite, alkali, or any other oxidizing agent), physically/mechanically degraded starch (e.g., via thermo-mechanical energy input of processing equipment), or a combination thereof.

In some examples, the active ingredient composition may include an anti-dandruff active. Suitable non-limiting examples of anti-dandruff actives include pyridinethione salts, azoles, selenium sulfide, particulate sulfur, keratolytic agents, and mixtures thereof.

In some examples, the active ingredient composition may include an opacifying agent. Opacifying agents are typically used in cleansing compositions to impart desired aesthetic benefits to hair care formulations, such as color or pearlescence. Suitable opacifying agents include, for example, fumed silica, polymethylmethacrylate, micronized TEFLON , boron nitride, barium sulfate, acrylate polymers, aluminum silicate, aluminum starch octenylsuccinate, calcium silicate, cellulose, chalk, corn starch, diatomaceous earth, Fuller's earth, glyceryl starch, hydrated silica, magnesium carbonate, magnesium hydroxide, magnesium oxide, magnesium trisilicate, maltodextrin, microcrystalline cellulose, rice starch, silica, titanium dioxide, zinc laurate, zinc myristate, zinc neodecanoate, zinc rosinate, zinc stearate, polyethylene, alumina, attapulgite, calcium carbonate, calcium silicate, dextran, nylon, silica silylate, silk powder, soy flour, tin oxide, titanium hydroxide, trimagnesium phosphate, walnut shell powder, or mixtures thereof. The above-mentioned powders may be surface treated with lecithin, amino acids, mineral oil, silicone oil, or various other agents either alone or in combination, which coat the powder surface and render the particles hydrophobic in nature.

In some examples, the active ingredient composition may include a suspending agent. Suspending agents useful herein include anionic polymers and nonionic polymers. Useful herein are vinyl polymers such as cross-linked acrylic acid polymers with the CTFA name Carbomer.

In some examples, the active ingredient composition may include a paraffinic hydrocarbon. Paraffinic hydrocarbons suitable for use in compositions of the present invention include those materials which are known for use in hair care or other personal care compositions, such as those having a vapor pressure at 1 atm of equal to or greater than about 21 C. (about 70 F.). Non-limiting examples include pentane and isopentane.

Active ingredient compositions of the present disclosure may contain foaming agents or foam boosters, such as ammonium lauryl sulfate, sodium lauryl sulfate, ammonium lauryl ether sulfate, and cocamidopropyl betaine. Active ingredient compositions of the present disclosure may include thickeners, such as xanthan gum and acrylate co-polymers. Active ingredient compositions of the present disclosure may also contain a mono- or divalent salt such as sodium chloride, chelating agents, and materials useful for hair loss prevention and hair growth stimulants or agents.

In some examples, the active ingredient composition may include water-soluble and water-insoluble vitamins such as vitamins B1, B2, B6, B12, C, pantothenic acid, pantothenyl ethyl ether, panthenol, biotin and their derivatives, and vitamins A, D, E, and their derivatives. The compositions of the present invention may also contain water-soluble and water-insoluble amino acids such as asparagine, alanine, indole, glutamic acid and their salts, and tyrosine, tryptamine, lysine, histidine, and their salts.

Additional active ingredient compositions including personal care active ingredients may include those materials approved for use in cosmetics and that are described in reference books such as the CTFA Cosmetic Ingredient Handbook, Second Edition, The Cosmetic, Toiletries, and Fragrance Association, Inc. 2016.

Preservatives, biocides, sanitizers, and antimicrobial agents as described herein may also be suitable for inclusion in an active ingredient composition of a disclosed dissolvable matrix or liquid precursor thereof. Other suitable agents are described, e.g., in US20150328115A1, US20090227675A1, CN201611013713.7A, CN201880013511.3A, ES15846461T, and US14/089,146.

Individual concentrations of such components may range from about 0.001 wt.% or greater, about 0.01 wt.% or greater, about 1 wt.% or greater, about 5 wt.% or greater, or about 6 wt.% or less, about 8 wt.% or less, or about 10 wt.% or less, or within any range using these endpoints, such as about 0.05 wt.% to about 5 wt.%, by weight of the dissolvable matrix composition.
The dissolvable matrix containing a personal active ingredient as described herein is free of polyvinyl alcohol (PVA).

### iii. Agricultural Active Ingredients

In some embodiments, disclosed dissolvable sheets contain an active ingredient composition that includes at least one agricultural active ingredient. In some embodiments, the active ingredient composition includes an agricultural active ingredient such as a fertilizer, pesticide, hormone, nutrient, or other treatment agents intended to improve the life or health or post-harvest condition of the agricultural product, or to ameliorate an adverse condition pertaining to the product, as would be understood by artisans of ordinary skill in the art.

In some embodiments, the active ingredient composition includes at least one pesticide. The term "pesticide," as used herein, is well known in the art and is described at least by the Environmental Protection Agency (EPA), in the Federal Insecticide, Fungicide, and Rodenticide Act (FIFRA), in the Insecticides and Environmental Pesticide Control Subchapter (7 U.S.C. § 136(u)), in the Code of Federal Regulations (CFR) relating to the "Protection of Environment," and in the Regulations of the EPA in 40 CFR § 152.3. A pesticide is typically recognized in the art as a substance that is used for preventing, destroying, repelling, regulating, and/or mitigating any pest. A pest is an organism that is deleterious to man or the environment but does not include any internal parasite of living man or other living animal or any fungus, bacterium, virus, or other microorganism on or in living man or other living animals. Said differently, the terminology "pest" does not typically include any organism that infects or sickens humans or animals. In addition, the terminology "pesticide," as used herein, does not typically include any human or animal drugs or pharmaceuticals, any article that is a "new animal drug" as defined in the art, any liquid sterilant applied to a device used in the human body, and/or any products intended for use against fungi, bacteria, viruses, or other microorganisms in or on living man or living animal. Moreover, the pesticide of this disclosure does not typically include drugs or pharmaceuticals used to control diseases of humans or animals (such as livestock and pets).

In some embodiments, the active ingredient composition includes at least one plant growth regulator. As used herein, the term "plant growth regulator" refers to a compound, which through physiological action will accelerate or retard the rate of growth or rate of maturation or otherwise alter the behavior of ornamental or crop plants or the products thereof.

Pesticides and plant growth regulators especially contemplated for use in the present invention are organic compounds, preferably synthetic organic compounds. Suitable pesticides and plant growth regulators include triazoles, strobilurins, alkylenebis(dithiocarbamate) compounds, benzimidazoles, phenoxy carboxylic acids, benzoic acids, ureas, sulfonylureas, triazines, pyridine carboxylic acids, neonicotinides, amidines, organophosphates, and pyrethroids. The pesticide may have a water solubility of 1g/L or less.

In some embodiments, the active ingredient composition includes additives, such as surfactants, water, thickeners, deposition enhancers, drift control agents, salts, stabilizers, penetrants, spreading agents, wetting agents, building agents, extending agents, emulsifiers, dispersants, suspending agents, plant penetrants, translocators, oils, activators, foliar nutrients, compatibility agents, drift retardants, foam retardants, buffers, inverting agents, soil penetrants, stabilizing agents, UV filters, feeding stimulants, washing agents, sinking agents, binders, liquid carriers, dry carriers such as attapulgite, kaolinite, vermiculite, starch polymers, corn cob, and combinations thereof. The pesticide formulation may also include additional chemical compounds that are not pesticides, such as activators, anti-feedants, anti-fouling agents, attractant agents, chemosterilants, disinfectant agents, fumigant agents, pheromones, repellent agents, defoliants, desiccants, insect growth regulators, plant growth regulators, synergists, adjuvants, and combinations thereof.

In some embodiments, the active ingredient composition includes a fungicide composition. Suitable fungicides include, but are not limited to: azoxystrobin, benalaxyl, carbendazim, chlorothalonil, cupfer, cymoxanil, cyproconazol, diphenoconazol, dinocap, epoxyconazol, fluazinam, flusilazol, flutriafol, folpel, fosetyl alumnium, kresoxim methyl, hexaconazol, mancozeb, metalaxyl, metconazol, myclobutanil, ofurace, phentinhydroxide, prochloraz, pyremethanil, soufre, tebucanazol and tetraconazol, and mixtures thereof.

In some embodiments, the active ingredient composition includes an herbicide composition. Suitable herbicides include, but are not limited to: alachlor, acloniphen, acetochlor, amidosulfuron, aminotriazol, atrazin, bentazon, biphenox, bromoxyl octanoate, bromoxynil, clethodim, chlodinafop-propargyl, chloridazon, chlorsulfuron, chlortoluron, clomazon, cycloxydim, desmedipham, dicamba, dicyclofop-methyl, diurea, difluphenicanil, dimithenamid, ethofumesat, fluazifop, fluazifop-p-butyl, fluorochloridon, fluroxypyr, glufosinat, glyphosate, galoxyfop-R, ioxynil octanoate, isoproturon, isoxaben, metamitron, metazachlor, metolachlor, metsulfuron-methyl, nicosulfuron, notflurazon, oryzalin, oxadiazon, oxyfluorphen, paraquat, pendimethalin, phenmedipham, phenoxyprop-p-ethyl, propaquizafop, prosulfocarb, quizalofop, sulcotrion, sulphosat, terbutylazin, triasulfuron, trichlorpyr, triflualin and triflusulforon-methyl which may be used individually or in admixture with one another.

In some examples, the fungicide composition may include other additives such as stabilizers, penetrants, spreading agents, wetting agents, building agents, extending agents, emulsifiers, dispersants, suspending agents, plant penetrants, translocators, oils, activators, foliar nutrients, compatibility agents, drift retardants, foam retardants, buffers, inverting agents, soil penetrants, stabilizing agents, UV filters, feeding stimulants, washing agents, sinking agents, binders, liquid carriers, dry carriers such as attapulgite, kaolinite, vermiculite, starch polymers, corn cob, and combinations thereof. The pesticide formulation may also include additional chemical compounds that are not pesticides, such as activators, anti-feedants, anti-fouling agents, attractant agents, chemosterilants, disinfectant agents, fumigant agents, pheromones, repellent agents, defoliants, desiccants, insect growth regulators, plant growth regulators, synergists, adjuvants, and combinations thereof.

As an example, a dissolvable matrix containing agricultural active ingredients can be used as a spray-on coating for plants or seeds. For living plants or plant materials, a dissolvable matrix can contain active agents intended to repel or kill insects, fungi, and the like, or can contain nutrients or other beneficial agents, or other agricultural active ingredients. For seeds, a dissolvable matrix coating can be used to mark or designate seeds, allowing color grading or other differentiation, or can repel moisture, dust, or other physical contaminants. Disclosed dissolvable matrix compositions can support active agents intended to enhance growth or protect against pests and fungi, including sustained release formulations of such active agents, and can furthermore protect against mechanical damage. In embodiments, the dissolvable matrix for seed coating can contain precisely formulated agents (such as fertilizers, micronutrients, crop protection chemicals and biologicals, temperature-sensitive polymers, water-retention materials, colorants, and beneficial organisms, etc.), which can be dispensed over appropriate time intervals following application. Dissolvable matrix compositions can further be formed into structures that can support preloaded seeds at precisely positioned spacings, with nutrients, fertilizers, or protective substances like weed-killers embedded within the matrices and optionally combined with materials that can retain water around the seed itself, thereby facilitating seed planting and optimizing seed growth.

For example, matrices comprising odoriferant materials can be readily formed from disclosed dissolvable matrix compositions, optionally in combination with plasticizers to control the release of the odoriferant. This technology can be adapted for agricultural purposes, for example with the use of pheromones as the agricultural active ingredients. Pheromones are understood to be secreted or excreted chemicals that trigger a social response in members of the same species. While they may not possess "odors" as the term is commonly understood, pheromone receptors are typically located in the olfactory epithelium or vomeronasal organ, indicating that they are processed by similar pathways as conventional. Pheromones are thus considered odor-related active agents for the purposes of the present disclosure.

Certain pheromones have use in the agricultural industry as pesticides or artificial growth hormones. Pheromones can be suspended in nanocellulose matrices and formed into sheets or formed articles, whereby the pheromones are released into the environment at a controlled rate as the nanocellulose matrix decomposes. The rate of release can be controlled through the careful selection of plasticizer and modification of the amount of it in the matrix substrate. Hydrophobicity can be controlled through the selection of an additive imparting hydrophobic properties to the matrix, such as a cellulosic polymer additive (e.g., a more or less hydrophobic polymer). Disclosed dissolvable matrix composition, containing more hydrophobic materials will take longer to break down, releasing the pheromones into the environment at a slower rate, encouraging long-term growth. An additional advantage of this technology is the contribution of the cellulose substrate to the soil, which encourages the activity of helpful insects in the environment to aerate the soil and break the cellulose down into useful materials such as carbon dioxide for the plants.

Other active agents having agricultural effects can be similarly incorporated into disclosed dissolvable matrix compositions for applications to growing plants. For example, in embodiments, growth hormones can be included in the matrices and released in a controlled manner, as described above. In other embodiments, insecticides, fungicides, pesticides, and the like can be incorporated into disclosed dissolvable matrix compositions. For these purposes, the matrix itself can include plasticizers to facilitate the diffusion of the active agents into the ambient air if this is the appropriate mechanism of action; such plasticizers can advantageously be hygroscopic to help the diffusion of the active agents if they are to be airborne. In order to hold the active agents within the nanocellulose matrix without premature release and without damaging the matrix itself, the matrix material can be thickened using a thickening agent such as cellulose polymers, starch, gelatin, or the like. Similar technology can be used for insect repellants, such as DEET, permethrin, picaridin, and the like. These materials can be incorporated into disclosed dissolvable matrix compositions, e.g., in sheet form, and applied directly to the skin or to articles of clothing. As the sheet dissolves, the residual active agent is deposited locally, continuing to provide insect repellent activity. Appropriate selection of plasticizers can prolong the release of the insect repellants, to prolong protection.

In addition, matrices used for agricultural purposes can be coated with or formulated with hydrophobic components to protect the agricultural active agents and prevent them from being washed off after application. This ability to protect the active agent and provide for controlled release can be useful for dispensing fertilizers to plants over a prolonged or predetermined period of time. By contrast, current fertilizer formulations are water-soluble, so that they can be easily washed away on rainy days. It is understood that a more hydrophobic selection of components for the disclosed dissolvable matrix compositions (promoted with the selection of more hydrophobic cellulose materials, such as methyl cellulose, or with the addition of oils or waxes) will take longer to break down in a moist environment, such as in the ground or in an area with high humidity, releasing the pesticides, hormones, fertilizers, etc., into the environment at a slower rate, encouraging long-term growth. An additional advantage of this technology is the contribution of cellulose to the soil, which encourages the activity of helpful insects in the environment to aerate the soil and break the cellulose down into useful materials such as carbon dioxide for the plants. The dissolvable matrix compositions disclosed herein can cooperate with the agricultural active agents to encourage healthy growth of plants for stronger, more abundant crops.

Individual concentrations of such components may range from about 0.001 wt.% or greater, about 0.01 wt.% or greater, about 1 wt.% or greater, about 5 wt.% or greater, or about 6 wt.% or less, about 8 wt.% or less, or about 10 wt.% or less, or within any range using these endpoints, such as about 0.05 wt.% to about 5 wt.%, by weight of the dissolvable matrix composition.

The dissolvable matrix compositions of the present disclosure have several advantageous properties. For example, the dissolvable matrix may contain a relatively low amount of an active ingredient composition. Relative to other carriers or matrix compositions, the superior dispersibility characteristics of disclosed matrix compositions allow for inclusion of reduced amounts of active ingredients in an active ingredient composition. For example, the active ingredient composition may have a total weight of from 0.1 to 2 grams (g), 0.1 to 1 g, 0.5 to 2 g, or 0.5 to 1 g.

Non-limiting examples of active ingredient compositions include laundry active ingredients, personal hygiene ingredients, and agricultural active ingredients. In some examples, the total weight of laundry active ingredients in a disclosed matrix may be as low as about 0.1 g, 0.2 g, 0.3 g, 0.4 g, 0.5 g, 0.6 g, 0.7 g, 0.8 g, 0.9 g, 1.0 g, or as high as 1.1 g, 1.2 g, 1.3 g, 1.4 g, 1.5 g, 1.6 g, 1.7 g, 1.8 g, 1.9 g, 2.0 g, 2.5 g, 3 g, 3.5 g, 4 g, or within any range encompassed by any two of the foregoing values as endpoints. In some examples, the total weight of personal hygiene ingredients in a disclosed matrix may be as low as about 0.1 g, 0.2 g, 0.3 g, 0.4 g, 0.5 g, 0.6 g, 0.7 g, 0.8 g, 0.9 g, 1.0 g, or as high as 1.1 g, 1.2 g, 1.3 g, 1.4 g, 1.5 g, 1.6 g, 1.7 g, 1.8 g, 1.9 g, 2.0 g, 2.5 g, 3 g, 3.5 g, 4 g, or within any range encompassed by any two of the foregoing values as endpoints. In some examples, the total weight of agricultural active ingredients in a disclosed matrix may be as low as about 0.1 g, 0.2 g, 0.3 g, 0.4 g, 0.5 g, 0.6 g, 0.7 g, 0.8 g, 0.9 g, 1.0 g, or as high as 1.1 g, 1.2 g, 1.3 g, 1.4 g, 1.5 g, 1.6 g, 1.7 g, 1.8 g, 1.9 g, 2.0 g, 2.5 g, 3 g, 3.5 g, 4 g, or within any range encompassed by any two of the foregoing values as endpoints.

The dissolvable matrix containing an agricultural active ingredient as described herein is free of polyvinyl alcohol (PVA).

### II. Liquid Formulations

Liquid formulations, also referred to as "liquid precursor" formulations or "wet mix" compositions are disclosed, but not according to the invention, such as liquid precursor solutions which can be processed and dried to form a dissolvable sheet as disclosed herein. Exemplary liquid formulations contain a cellulose substrate, a cellulose derivative, and an active ingredient composition in a liquid medium, such as water.

Liquid formulations not according to the invention contain cellulose fibers.
Depending on the size of the fibers, liquid formulations can contain cellulose nanofibers (nanocellulose), cellulose microfibers (microfibrillated cellulose), or a combination thereof. Cellulose nanofibers and cellulose microfibers can be distinguished from each other based on their size and shape. Cellulose nanofibers, which are also referred to as nanofibrillated cellulose have a smaller diameter than cellulose microfibers. Also referred to as microfibrillated cellulose, cellulose microfibers can be straight and rod-like or more flexible in appearance or irregular in shape. While the literature cites a range of dimensions for cellulose nanofibers and for cellulose microfibers, nanocellulose fibers are nanoscale, e.g., having a diameter between 4-20 nm, while CMF can be much larger. However, cellulose microfibers fibers typically still have diameters in the nano-range, e.g., 20-100 nm or larger.

As noted herein, reference to a nanocellulose material does not exclude the presence of cellulose fiber that exceeds nanoscale. For example, a nanocellulose substrate may also include micron-scale cellulose fibers. In some examples, a cellulose substrate for inclusion in the liquid formulation has a D10 value ranging from 1 µm to 35 µm, 5 µm to 35 µm, 10 µm to 35 µm, 25 µm to 35 µm, 1 µm to 25 µm, 5 µm to 25 µm, 10 µm to 25 µm, or 15 µm to 25 µm; a D50 value ranging from 50 µm to 150 µm, 75 µm to 150 µm, 100 µm to 150 µm, 50 µm to 125 µm, 75 µm to 125 µm, 100 µm to 125 µm, 50 µm to105 µm, 75 µm to 105 µm, or 100 µm to 105 µm; and a D90 value ranging from 350 µm to 950 µm, 550 µm to 950 µm, 750 µm to 950 µm, 250 µm to 750 µm, 450 µm to 750 µm, 600 µm to 750 µm, 400 µm to 600 µm, 450 µm to 600 µm, 500 µm to 600 µm, or 550 µm to 600 µm, wherein each range is inclusive.

Disclosed liquid formulations not according to the invention can include a cellulose substrate, such as nanocellulose, a cellulose derivative, and an active ingredient composition as described herein. In other examples, disclosed liquid formulations include a cellulose substrate and a cellulose derivative, but not an active ingredient composition. The active ingredient composition may include a laundry active ingredient, a personal care active ingredient, an agricultural active ingredient, or a combination thereof.

Disclosed liquid formulations not according to the invention include cellulose, a cellulose derivative, and an active ingredient composition that includes a surfactant, a starch, a dispersion aid, and an enzyme blend. In additional preferred examples, disclosed liquid formulations further include a fragrance.

Disclosed liquid formulations not according to the invention include a cellulose substrate in an amount of 25-40 wt%, 30-50 wt%, or 30-40 wt% relative to the total weight of the liquid formulation. The cellulose substrate can include nanocellulose, microfibrillated cellulose, or a combination thereof.

In some examples, the liquid formulation not according to the invention contains a cellulose derivative selected from hydroxyethyl cellulose (HEC), hydroxypropyl cellulose (HPC), hydroxypropyl methylcellulose (HPMC), methyl cellulose, carboxymethylcellulose (CMC), or a combination thereof. In other examples, the liquid formulation contains HEC, HPC, HPMC, methyl cellulose, or CMC. In preferred examples, the liquid formulation not according to the invention contains CMC. The liquid formulation may contain the cellulose derivative in a total amount of about 0.1-5 wt%, 0.1-10 wt%, 0.1-15 wt%, 1-5 wt%, 1-10 wt%, or 1-15 wt%, relative to the total weight of the liquid formulation. In some examples, the liquid formulation not according to the invention contains the cellulose derivative in a total amount of about 1 wt%, 2 wt%, 3 wt%, 4 wt%, 5 wt%, 6 wt%, 7 wt%, 8 wt%, 9 wt%, or 10 wt% relative to the total weight of the liquid formulation.

Liquid formulations include surfactants. In preferred examples, the liquid solution not according to the invention includes an anionic surfactant, such as SLES, in combination with a nonionic surfactant. In some embodiments, the liquid formulation contains one or more surfactants in a total amount of 1-30 wt %, 5-30 wt %, 10-30 wt %,1-25 wt %, 5-25 wt %, 10-25 wt %, 15-25 wt %, 20-25 wt %, 1-20 wt %, 5-20 wt %, 10-20 wt %, 15-20 wt %, 5-15 wt %, 10-15 wt %, 1-10 wt %, 5-10 wt %, or 7-15 wt % relative to the total weight of the liquid formulation.

In some embodiments, the liquid formulation not according to the invention contains an active ingredient composition that is present in a total amount that is less than 30 wt%, less than 20 wt%, less than 15 wt%, less than 10 wt%, less than 5 wt%, or less than 1 wt% relative to the total weight of the liquid formulation. In some embodiments, the liquid formulation not according to the invention contains an active ingredient composition that is present in a total amount that is 0.1-30 wt%, 0.5-30 wt%, 1-30 wt%, 5-30 wt%, 10-30 wt%, 0.5-25 wt%, 1-25 wt%, 5-25 wt%, 10-25 wt%, 15-25 wt%, 20-25 wt%, 0.1-20 wt%, 0.5-20 wt%, 1-20 wt%, 5-20 wt%, 10-20 wt%, 15-20 wt%, 5-15 wt%, 10-15 wt%, or 7-9 wt% relative to the total weight of the liquid formulation. In some embodiments, the active ingredient composition includes a laundry active ingredient, a personal care active ingredient, an agricultural active ingredient, or a combination thereof.

In some embodiments, the active ingredient composition contains a dispersion aid. In some embodiments, the dispersion aid is present in a total amount of 10% or less, 7% or less, 5% or less, 3% or less, or 1% or less relative to the total weight of the liquid formulation. In some embodiments, the dispersion aid is present in a total amount of 1-15 wt%, 5-15 wt%, 10-15 wt%, 1-10 wt%, 1-5 wt%, or 2-5 wt% relative to the total weight of the liquid formulation. In preferred examples, the dispersion aid is selected from polyethylene glycol, poly-glycols, mono or polyhydric alcohols, water-miscible glycols, glycol ethers, polyols, triols, sugar alcohols, and combinations thereof.

The active ingredient composition contains a starch. In some embodiments, the starch is present in a total amount of 7 wt% or less, 5 wt% or less, 3 wt% or less, or 1 wt% or less relative to the total weight of the liquid formulation. In some embodiments, the starch is present in a total amount of 11-5 wt%, or 2-5 wt% relative to the total weight of the liquid formulation. In preferred examples, the starch is a modified starch.

The active ingredient composition contains an enzyme blend. In some embodiments, the enzyme blend is present in a total amount of 7 wt% or less, 5 wt% or less, 3 wt% or less, or 1wt% or less relative to the total weight of the liquid formulation. In some embodiments, the enzyme blend is present in a total amount of 1-5 wt%, or 2-5 wt% relative to the total weight of the liquid formulation. In some embodiments, the enzyme blend is present in a total amount of relative to the total weight of the liquid formulation. In preferred examples, the active composition contains an enzyme blend including a protease enzyme, an amylase enzyme, a pectate lyase enzyme, a mannanase enzyme, a lipase enzyme, or a combination thereof.

The active ingredient composition contains a preservative. In some embodiments, the preservative is present in a total amount 0.25 wt% or less, 0.1 wt% or less, or 0.05 wt% or less relative to the total weight of the liquid formulation. In some embodiments, the preservative is present in a total amount of 0.05-0.5 wt%, or 0.05-0.25 wt% relative to the total weight of the liquid formulation.

In some embodiments, the active ingredient composition contains a fragrance. In some embodiments, the fragrance is present in a total amount of 1 wt% or less, 0.75 wt% or less, 0.5 wt% or less, 0.25 wt% or less, 0.1 wt% or less, or 0.05 wt% or less relative to the total weight of the liquid formulation In some embodiments, the fragrance is present in a total amount of 0.1-5 wt%, 0.5-5 wt%, 1-5 wt%, 1-3 wt%, 3-5 wt%, or 2-4 wt% relative to the total weight of the liquid formulation.

In some embodiments, the ingredients of the liquid precursor formulation are mixed in water. As described in the preceding section, such liquid formulations can be processed, such as exposed to heat and/or electromagnetic radiation, to form a dried substrate which includes any of a variety of active agents. The dried substrate can be formed, shaped, or cut into any of a number of configurations, including square, rectangular, circular, or diamond shapes as well as sheets, such as a single sheet or a stacked or layered sheet.

### III. Methods of Manufacturing Dissolvable Matrix Compositions

Also provided are methods for producing the dissolvable matrix composition of the present disclosure. An exemplary method for manufacturing a dissolvable sheet includes the following steps:
(i) mixing together the nanocellulose substrate, a cellulose derivative, and the active ingredient composition, thereby producing a liquid precursor formulation; and
(ii) drying the liquid precursor formulation, thereby producing a dissolvable matrix composition; wherein the drying step reduces the water content of the liquid precursor formulation by at least 35%, 45%, 55%, 65%, or 75%.

In some embodiments, disclosed methods of manufacturing also include (iii) shaping the dissolvable matrix composition into a desired orientation, such as a sheet.

In some embodiments, the drying step is repeated using a variety of different drying techniques and combinations thereof. For example, the liquid precursor formulation can be dried in an oven, a drum, such as a heated rotating drum, or exposed to electromagnetic radiation, e.g., infrared heating. In other embodiments, the liquid precursor formulation can be spray dried or freeze-dried (lyophilized). In additional embodiments, the liquid precursor formulation can be dried by supercritical CO₂ drying. Other suitable drying techniques will be recognized to one of skill in the art based on several factors including the desired properties of the disclosed dissolvable matrix, the scale of production, and the sensitivity, such as the temperature sensitivity of dissolvable matrix components, including the cellulose substrate, the cellulose derivative, and optionally the active ingredient composition.

In other embodiments, the drying step involves applying a dewatering techniques to the liquid precursor formulation. Whereas drying involves removing water by evaporation, dewatering involves applying a significant mechanical force to the liquid precursor formulation to remove water.

In some embodiments, the active ingredient composition is added to the wet mix or liquid precursor formulation for inclusion in the final dried dissolvable matrix. In other embodiments, an active ingredient composition may be sprayed or coated onto a disclosed dissolvable matrix. In some embodiments, manufacturing disclosed dissolvable matrix compositions primarily involves drying a liquid precursor formulation into a dried dissolvable matrix. In other embodiments, manufacturing disclosed dissolvable matrix compositions primarily involves spraying, coating, or submerging the dissolvable matrix composition into an active ingredient composition. In other embodiments, manufacturing disclosed dissolvable matrix compositions primarily involves processing a disclosed dissolvable matrix into a desired shape or geometry, e.g., cutting the dried dissolvable matrix composition to length to form sheets of the dissolvable matrix compositions.

An exemplary method for manufacturing a dissolvable sheet includes the following steps:
(i) mixing an active ingredient composition with a nanocellulose fiber to form a liquid precursor solution,
(ii) processing said solution through a roll coater onto a slip sheet,
(iii) passing the slip sheet through a heater to form a sheet roll, and
(iv) drying and cutting the sheet roll into individual dissolvable sheets.

Manufacturing a wet mix solution involves adding ingredients, such as nanocellulose, water, and active agents, to a vessel and mixing at various speeds to ensure uniform dispersion of the ingredients in solution. The wet mix can then be processed through a doctor blade roll coater or other suitable equipment onto a slip sheet through a convection oven at a speed (meters/minute) based on drying requirements.

The speed of the slip sheet through the convection oven may be as low as 0.1 meters per minute, 0.5 meters per minute, 1 meter per minute, or as high as 1.5 meters per minute, 2 meters per minute, 2.5 meters per minute, or within any range encompassed by any two of the foregoing values as endpoints. For example, the slip sheet speed through the convection oven may be from 0.5 meters per minute to 1 meter per minute, 0.5 meters per minute to 1.5 meters per minute, or 0.5 meters per minute to 2 meters per minute, wherein each range is inclusive.

The heater may be a convection oven or any other suitable heater. The heater may be configured to provide heating from the top and bottom sides of the sheet roll simultaneously.

The heater may be configured to allow the internal temperature of the sheet roll to fall within a specific range which facilitates drying of the substrate and prevents other ingredients, such as active agents, from evaporating or reaching their flash point. Throughout the drying process, the highest temperature reached by the inside of the sheet roll, such as the maximum internal temperature of the sheet roll, may be less than 38°C, 41°C, 43°C, 46°C, 49°C, 52°C, 54°C, 57°C, 60°C, 63°C, 66°C (100°F, 105°F, 110°F, 115°F, 120°F, 125°F, 130°F, 135°F, 140°F, 145°F, 150°F), or within any range encompassed by any two of the foregoing values as endpoints. In other examples, the maximum internal temperature of the sheet roll may be about 38°C, 41°C, 43°C, 46°C, 49°C, 52°C, 54°C, 57°C, 60°C, 63°C, 66°C (100°F, 105°F, 110°F, 115°F, 120°F, 125°F, 130°F, 135°F, 140°F, 145°F, 150°F), or within any range encompassed by any two of the foregoing values as endpoints. In preferred examples, the maximum internal temperature of the sheet roll is less than or equal to 49°C (120°F), e.g., in the range of about 38-43°C, 38-46°C, 38-49°C, 41-43°C, 41-46°C, 41-49°C, 43-46°C or 43-49°C (100°F to 110°F, 100°F to 115°F, 100°F to 120°F, 105°F to 110°F, 105°F to 115°F, 105°F to 120°F, 110°F to 115°F, or 110°F to 120°F), wherein each range is inclusive.

The heater's temperature may be as low as 38°C, 43°C, 49°C, 54°C, 60°C (100°F, 110°F, 120°F, 130°F, 140°F), or as high as 66°C, 68°C, 71°C, 77°C, 82°C, 88°C, 93°C, 107°C, 121°C, 135°C, 149°C (150°F, 155°F, 160°F, 170°F, 180°F, 190°F, 200°F, 225°F, 250°F, 275°F, 300°F), or within any range encompassed by any two of the foregoing values as endpoints. For example, the heater temperature may be from 60-71°C, 60-77°C, 66-71°C, 66-77°C, 66-93°C, 66-107°C, 66-121°C, 66-135°C, 66-149°C, 93-149°C, 99-143°C, 99-121°C, 121-143°C (140°F to 160°F, 140°F to 170°F, 150°F to 160°F, 150°F to 170°F, 150°F to 200°F, 150°F to 225°F, 150°F to 250°F, 150°F to 275°F, 150°F to 300°F, 200°F to 300°F, 210°F to 290°F, 210°F to 250°F, or 250°F to 290°F), wherein each range is inclusive.

In some embodiments, the sheet roll can be subjected to an additional drying step after it exits or is removed from the oven. For example, the sheet roll can be exposed to heat via electromagnetic radiation, e.g., infrared heating. Regardless of the number of drying steps or exposure to heat, it is preferred for the maximum internal temperature of the sheet roll not to exceed 49°C (120°F).

The preceding disclosure should not be construed as limiting, especially as it relates to processing equipment, such as coaters, slip sheets, heat sources, and equipment for shaping, molding, or otherwise forming a disclosed dissolvable matrix composition into a desired shape. Suitable alternatives for one or more steps in manufacturing of a dissolvable matrix may be contemplated by one of ordinary skill in the art.

For example, disclosed dissolvable matrix compositions can be shaped, molded, or cut into a desired geometry, such as a chip, a strip, a ball, a pod, a cube, or a sheets, such as a single layer or multi-layer sheets. Shape can be adapted to the specifications of a conceivable product, e.g., a laundry article, an in-scent booster article, an odor neutralizing article, a fabric softener article, an all-purpose cleaning article, a toilet bowl cleaning article, a bathroom cleaning article, a dishwashing article, or an auto dishwash article.

By way of non-limiting illustration, examples of certain embodiments of the present disclosure are given below.

### EXAMPLES

### Example 1: Dispersion Performance of Laundry Sheets

In this test, an exemplary sheet according to the disclosure (Example 1) and a comparison sheet (Comparative Example 1) were dissolved in water to test their dispersion performance. The exemplary sheet was composed of a cellulose substrate, a cellulose derivative, and an active ingredient composition including laundry active ingredients. Physical dimensions of the sheets are provided in Table 1.

**Table 1. Comparative Laundry Sheet Compositions and Dimensions**

| (1 inch =25.4mm throughout) | | |
|---|---|---|
| **Example** | **Laundry Sheet** | **Dimensions** |
| Example 1 | Nanocellulose laundry sheet | 1 and 1/8 inch by 1 and 1/2 inch |
| Comparative Example 1 | National Brand PVA laundry sheet | 1 and 1/8 inch by 1 and 1/2 inch |

A beaker filled with 1600 ml of water at 69°C was placed on a magnetic stir plate. The dimensions of the exemplary and comparison sheets and the volume of water were calculated based on the concentration produced with a typical 102mm and 51mm by 152mm (4 and 1/2 inch by 6 inch) sheet with a medium load of laundry in a front end loader's cleaning cycle. A four pronged magnetic stir bar was added, and the stirring was set at 630 rpm. Example 1 or Comparative Example 1 was added to the beaker and the mixture was stirred for 5 minutes. After 5 minutes, the stirring was stopped, and the stir bar was removed. An image of the liquid mixture was taken. A clear appearance indicated solubilization of the ingredients. A cloudy appearance indicated poor solubilization. Fig. 1A corresponds to Example 1 and Fig. 1B corresponds to Comparative Example 1. The results indicate that the inventive Example had improved solubilization relative to the comparison sheet.

### Reference Example 2: Varying the CMC Concentration of a Wet Mix Formulations Alters Viscosity, and Impacts Sheet Formation, and Enhances Dispersibility

Properties of exemplary wet mix compositions containing various amounts of CMC were assessed, including viscosity, sheet formation, and sheet dispersibility. Exemplary wet mix compositions contained nanocellulose, CMC, alcohol, SLES, starch, an enzyme blend, and water. The amount of starch was adjusted to compensate for a decreased level of CMC or the absence of CMC.

Viscosity was measured for wet mix formulations containing 0% CMC, 1.2% CMC, and 3.1% CMC according to standard procedures. Briefly, the slurries were tested at room temperature (65°F) with a Cole- Parmer digital viscometer with spindles L3 & L4 at various RPMs for percentages of acceptable accuracy. The viscosity (centipoise/cP), spindle number, RPM, and accuracy (%) were recorded. As shown in Table 2, the viscosity of the wet mix without CMC (0% CMC) was significantly lower than that of the other wet mixes. The 0% CMC wet mix lacked the cohesion to form a sheet.

**Table 2. Comparative Viscosity of Wet Mix Formulations Containing 0%, 1.24%, and 3.11% CMC**

| (wherein 1cP=0.001mPa.s) | | |
|---|---|---|
| **0% CMC** | **1.24% CMC** | **3.11% CMC** |
| 14,752 cP | 26,356 cP | 48,323 cP |
| (L3 Spindle - 6 RPM - 73.8% Accuracy) | (L4 Spindle - 20 RPM - 87.9% Accuracy) | (L4 Spindle - 12 RPM - 96.7% Accuracy) |

While the viscosity of the 0% CMC wet mix was insufficient for sheet formation, wet mixes containing 1.2% and 3.1% CMC were successfully formed and dried into sheets. Following sheet formation, comparative dispersibility was assessed by submerging the sheet into a beaker with a stir bar and purified water at a temperature of 65°F (18°C). The speed was set to 6-20 RPM, as provided in Table 2, and dispersibility was judged by eye after 5 minutes.

The sheet containing 3.1% CMC dispersed to a greater extent than the sheet containing less CMC. After the 5 minute dispersion experiment, a sizeable portion of the sheet remained intact, whereas the sheet containing 3.1% CMC was almost completely solubilized, as evidenced by clarity of the solution and the absence of intact sheet portions. Together, the results highlight the surprising role of CMC both as a cohesive agent in sheet formation and also as an enhancer of sheet dispersibility.

### Reference Example 3: Exemplary Method of Manufacturing a Disclosed Sheet

The following methods describe an exemplary method of manufacturing a substrate matrix with at least one active agent in accordance with the present disclosure, a laundry sheet that is free from PVA, dissolves easily and cleans stains.

To prepare the wet mix formulation, deionized water and ethoxylated alcohol were added to a mixing vessel and mixed at 400 RPM. Starch was then added. After the starch dispersed in the mixture, CMC was slowly added while increasing the RPM. Isopropyl alcohol was then added to the mixture along with nanofibrillated cellulose (NFC) and sodium lauryl ether sulfate (SLES). Both NFC and SLES were dispersed evenly to produce a uniform mixture. The remaining ingredients were added, including a fragrance active agent, and mixing continued.

The mixture was processed through a doctor blade roll coater onto a slip sheet through a convection oven with controlled temperature heaters at the top and bottom. The internal temperature of the product sheet was not allowed to reach or exceed 120°F (approximately 49°C) to prevent sheet components from reaching their flash point. After drying, the sheet web was wound on a mandrel and slit to width. The slit rolls were processed through a rotary sheeter, stacked, and packed into a box for storage and distribution.

### Example 4: Laundry Sheet

A dissolvable matrix composition as described herein containing an active ingredient composition with a laundry active ingredient is added to a washing machine containing clothes for washing. The laundry sheet does not need to be torn as it will dissolve in the wash water. The appropriate wash cycle and temperature is chosen. Use of the laundry sheet as disclosed herein obviates the need for messy measuring and pouring of liquid detergent. It also avoids the detrimental environmental impact associated with PVA-containing detergent delivery systems, such as laundry pods. Once the wash cycle is complete, clothes are removed from the washing machine and any of effective cleaning, stain lifting, freshening, and combinations thereof are observed.

### Example 5: Personal Care Sheet

A dissolvable matrix composition as described herein containing an active ingredient composition with a personal care active ingredient. The sheet is moistened, e.g., wet with water, and gently massaged onto the desired area for personal care or cleansing, such as the skin or hair. Once the desired lather is produced, such as from dispersal of the personal care active ingredients following contact with water, the area of application may be rinsed with water. The pre-measured amount of personal care ingredients in each sheet avoids excessive use and potential waste of the personal care ingredients. The sheet is compact and lightweight, which advantageously facilitate portability in any of several contexts, including camping, travel, and gym use.

### Example 6: Agricultural Sheet

A dissolvable matrix composition as described herein containing an active ingredient composition with an agricultural active ingredient. The agricultural sheet may be used to suppress weed growth, conserve moisture, or regulate soil temperature. The agricultural sheet may also be used as a seed mat or a germination blanket. Such sheet compositions are biodegradable and may contain ingredients, including fertilizer and other soil additives, to promote seed germination and growth.

## Claims

1. A sheet, wherein the sheet is dissolvable, the sheet comprising:
a non-woven web comprising a nanocellulose substrate, a chemically modified version of cellulose being either a cellulose ether or a cellulose ester, and an active ingredient composition,
**characterized in that** the sheet comprises:
25 wt.% to 55 wt.% cellulose;
10 wt.% to 45 wt.% of a nonionic surfactant;
1 wt.% to 10 wt.% of an anionic surfactant;
1 wt.% to 15 wt.% of the chemically modified version of cellulose;
1 wt.% to 10 wt.% of glycerine;
0.1 wt.% to 8 wt.% of an enzyme blend;
0.5 wt.% to 8 wt.% of a starch;
0.01 wt.% to 0.5 wt.% of a preservative; and
1-20 wt.% water, relative to the total weight of the dissolvable sheet,
and wherein the dissolvable sheet does not contain polyvinyl alcohol (PVA).

2. The sheet of either claim 1 , wherein the chemically modified version of cellulose is hydroxyethyl cellulose (HEC), hydroxypropyl cellulose (HPC), hydroxypropyl methylcellulose (HPMC), methyl cellulose, or carboxymethylcellulose sodium (NaCMC).

3. The sheet of claim 2, wherein the chemically modified version of cellulose is NaCMC, and wherein the CMC is present in an amount of 1 to 15 wt.% relative to the total weight of the sheet.

4. The sheet of claim 1, wherein the enzyme blend comprises at least one protease enzyme, at least one amylase enzyme, at least one pectate lyase enzyme, at least one mannanase enzyme, at least one lipase enzyme, or a combination thereof.

5. The sheet of any of the preceding claims, wherein the sheet has a weight of about 1 gram to 8 grams, wherein the range is inclusive.

6. The sheet of any of the preceding claims, wherein the sheet has a thickness of about 0.1 to 0.3 millimeters, wherein the range is inclusive.

7. The sheet of claim 2, wherein the nanocellulose substrate comprises a nanocellulose fiber which is naturally derived from softwood, hardwood, soy hulls, wheat straw, bagasse, sugar beet pulp, or a combination thereof.

8. The sheet of any of the preceding claims, wherein the active ingredient comprises one of a cleaning product, a laundry detergent, a surfactant, a dishwasher soap, a hand soap, a skin or makeup cleanser, a fabric softener, an agricultural composition, a dermatological composition, a pharmaceutical composition, a cosmetic composition, or a combination of any of the foregoing.

9. The sheet of any one of the preceding claims, wherein the active ingredient comprises:
at least one of scent boosters, static reducers, stain removal agents, anti-redeposition agents, degreasing agents, bleaching agents, whitening agents, fluorescent whitening agents, brighteners, dye transfer inhibiting agents, chlorine scavengers, chelating agents, corrosion inhibitors, opacifiers, suds suppressors, suds boosters, conditioning agents, colorants, dyes, fabric enhancing polymers, pH adjusters, buffering agents, antibacterials, antimicrobials, malodor control agents, and perfumes.

10. The sheet of any one of the proceeding claims, wherein the active ingredient comprises:
at least one of shampoo, conditioner, body wash, facial cleanser, moisturizers, makeup remover, deodorant, sanitizers, antibacterials, sunscreen, and ingredients for pet grooming.

11. A method of manufacturing a sheet as defined in claim 1, comprising:
(i) mixing the nanocellulose substrate, the chemically modified version of cellulose, and the active ingredient composition together, thereby producing a liquid precursor formulation; and
(ii) drying the liquid precursor formulation, thereby producing a dissolvable matrix composition, wherein the drying step reduces the water content of the liquid precursor formulation by at least 35%.

12. A method of manufacturing a sheet as defined in claim 1 comprising:
(i) mixing the nanocellulose substrate, the chemically modified version of cellulose, and the active ingredient composition together, thereby producing a liquid precursor formulation;
(ii) processing the liquid precursor formulation through a roll coater onto a slip sheet;
(iii) drying the liquid formulation by passing the slip sheet through a heater to form a dried sheet roll; and
(iv) cutting the dried sheet roll into individual sheets.

13. The method of claim 12, wherein the heater is a convection oven.

14. The method of either claim 12 or 13, wherein the slip sheet passes through the heater at a speed of from about 0.5 meters per minute to 1.5 meters per minute.

15. The method of any of claims 12 - 14, wherein the liquid formulation is not heated above 49°C (120°F) and the maximum internal temperature of the sheet roll does not exceed 49°C (120°F).

## Patentansprüche

1. Ein Blatt, wobei das Blatt auflösbar ist, wobei das Blatt Folgendes beinhaltet:
ein Vliesnetz, das ein Nanocellulosesubstrat, eine chemisch modifizierte Version von Cellulose, die entweder ein Celluloseether oder ein Celluloseester ist, und eine Wirkstoffzusammensetzung beinhaltet,
**dadurch gekennzeichnet, dass** das Blatt Folgendes beinhaltet:
zu 25 Gew.-% bis 55 Gew.-% Cellulose;
zu 10 Gew.-% bis 45 Gew.-% ein nichtionisches Tensid;
zu 1 Gew.-% bis 10 Gew.-% ein anionisches Tensid;
zu 1 Gew.-% bis 15 Gew.-% die chemisch modifizierte Version von Cellulose;
zu 1 Gew.-% bis 10 Gew.-% Glycerin;
zu 0,1 Gew.-% bis 8 Gew.-% eine Enzymmischung;
zu 0,5 Gew.-% bis 8 Gew.-% eine Stärke;
zu 0,01 Gew.-% bis 0,5 Gew.-% ein Konservierungsmittel; und
zu 1-20 Gew.-% Wasser, bezogen auf das Gesamtgewicht des auflösbaren Blatts,
und wobei das auflösbare Blatt keinen Polyvinylalkohol (PVA) enthält.

2. Blatt gemäß Anspruch 1, wobei die chemisch modifizierte Version von Cellulose Hydroxyethylcellulose (HEC), Hydroxypropylcellulose (HPC), Hydroxypropylmethylcellulose (HPMC), Methylcellulose oder Natriumcarboxymethylcellulose (NaCMC) ist.

3. Blatt gemäß Anspruch 2, wobei die chemisch modifizierte Version von Cellulose NaCMC ist und wobei das CMC in einer Menge von 1 bis 15 Gew.-%, bezogen auf das Gesamtgewicht des Blatts, vorhanden ist.

4. Blatt gemäß Anspruch 1, wobei die Enzymmischung mindestens ein Proteaseenzym, mindestens ein Amylaseenzym, mindestens ein Pektatlyaseenzym, mindestens ein Mannanaseenzym, mindestens ein Lipaseenzym oder eine Kombination davon beinhaltet.

5. Blatt gemäß einem der vorstehenden Ansprüche, wobei das Blatt ein Gewicht von etwa 1 Gramm bis 8 Gramm aufweist, wobei der Bereich einschließlich ist.

6. Blatt gemäß einem der vorstehenden Ansprüche, wobei das Blatt eine Dicke von etwa 0,1 bis 0,3 Millimeter aufweist, wobei der Bereich einschließlich ist.

7. Blatt gemäß Anspruch 2, wobei das Nanocellulosesubstrat eine Nanocellulosefaser beinhaltet, die natürlicherweise von Nadelholz, Hartholz, Sojaschalen, Weizenstroh, Bagasse, Zuckerrübenschnitzel oder einer Kombination davon abgeleitet ist.

8. Blatt gemäß einem der vorstehenden Ansprüche, wobei der Wirkstoff eines von einem Reinigungsprodukt, einem Waschmittel, einem Tensid, einer Geschirrspülerseife, einer Handseife, einem Haut- oder Make-up-Reiniger, einem Weichspüler, einer landwirtschaftlichen Zusammensetzung, einer dermatologischen Zusammensetzung, einer pharmazeutischen Zusammensetzung, einer kosmetischen Zusammensetzung oder einer Kombination von beliebigen der Vorstehenden beinhaltet.

9. Blatt gemäß einem der vorstehenden Ansprüche, wobei der Wirkstoff Folgendes beinhaltet:
mindestens eines von Duftverstärkern, statischen Reduktionsmitteln, Fleckenentfernungsmitteln, Antiredepositionsmitteln, Entfettungsmitteln, Bleichmitteln, Aufhellern, fluoreszierenden Aufhellern, Glanzmitteln, Farbstoffübertragungshemmern, Chlorfängern, Chelatbildnern, Korrosionsinhibitoren, Trübungsmitteln, Schaumunterdrückern, Schaumverstärkern, Konditionierungsmitteln, Farbstoffen, Färbemitteln, textilverstärkenden Polymeren, pH-Einstellmitteln, Puffermitteln, antibakteriellen Mitteln, antimikrobiellen Mitteln, Mitteln zur Bekämpfung von schlechtem Geruch und Duftstoffen.

10. Blatt gemäß einem der vorstehenden Ansprüche, wobei der Wirkstoff Folgendes beinhaltet:
mindestens eines von Shampoo, Konditionierer, Körperspülung, Gesichtsreiniger, Feuchtigkeitsspendern, Make-up-Entferner, Deodorant, Desinfektionsmitteln, antibakteriellen Mitteln, Sonnenschutzmitteln und Inhaltsstoffen zum Pflegen von Haustieren.

11. Ein Verfahren zur Herstellung eines Blatts gemäß Anspruch 1, das Folgendes beinhaltet:
(i) Zusammenmischen des Nanocellulosesubstrats, der chemisch modifizierten Version von Cellulose und der Wirkstoffzusammensetzung, wodurch eine flüssige Vorläuferformulierung produziert wird; und
(ii) Trocknen der flüssigen Vorläuferformulierung, wodurch eine auflösbare Matrixzusammensetzung produziert wird, wobei der Trocknungsschritt den Wassergehalt der flüssigen Vorläuferformulierung um mindestens 35 % verringert.

12. Ein Verfahren zur Herstellung eines Blatts gemäß Anspruch 1, das Folgendes beinhaltet:
(i) Zusammenmischen des Nanocellulosesubstrats, der chemisch modifizierten Version von Cellulose und der Wirkstoffzusammensetzung, wodurch eine flüssige Vorläuferformulierung hergestellt wird;
(ii) Verarbeiten der flüssigen Vorläuferformulierung durch einen Walzenbeschichter auf einen Einschussbogen;
(iii) Trocknen der flüssigen Formulierung durch Leiten des Einschussbogens durch eine Heizvorrichtung, um eine getrocknete Blattrolle zu bilden; und
(iv) Schneiden der getrockneten Blattrolle in einzelne Blätter.

13. Verfahren gemäß Anspruch 12, wobei die Heizvorrichtung ein Konvektionsofen ist.

14. Verfahren gemäß Anspruch 12 oder 13, wobei der Einschussbogen mit einer Geschwindigkeit von etwa 0,5 Metern pro Minute bis 1,5 Metern pro Minute durch die Heizvorrichtung geleitet wird.

15. Verfahren gemäß einem der Ansprüche 12-14, wobei die flüssige Formulierung nicht über 49 °C (120 °F) erhitzt wird und die maximale Innentemperatur der Blattrolle 49 °C (120 °F) nicht überschreitet.

## Revendications

1. Une feuille, la feuille étant soluble, la feuille comprenant :
une toile non tissée comprenant un substrat de nanocellulose, une version chimiquement modifiée de cellulose qui est soit un éther de cellulose soit un ester de cellulose, et une composition d'ingrédients actifs,
**caractérisée en ce que** la feuille comprend :
de 25 % en poids à 55 % en poids de cellulose ;
de 10 % en poids à 45 % en poids d'un agent tensioactif non ionique ;
de 1 % en poids à 10 % en poids d'un agent tensioactif anionique ;
de 1 % en poids à 15 % en poids de la version chimiquement modifiée de cellulose ;
de 1 % en poids à 10 % en poids de glycérine ;
de 0,1 % en poids à 8 % en poids d'un mélange homogène d'enzymes ;
de 0,5 % en poids à 8 % en poids d'un amidon ;
de 0,01 % en poids à 0,5 % en poids d'un conservateur ; et
1-20 % en poids d'eau, par rapport au poids total de la feuille soluble,
et où la feuille soluble ne contient pas d'alcool polyvinylique (PVA).

2. La feuille de la revendication 1, où la version chimiquement modifiée de cellulose est l'hydroxyéthylcellulose (HEC), l'hydroxypropylcellulose (HPC), l'hydroxypropylméthylcellulose (HPMC), la méthylcellulose, ou la carboxyméthylcellulose sodique (NaCMC).

3. La feuille de la revendication 2, où la version chimiquement modifiée de cellulose est la NaCMC, et où la CMC est présente en une quantité allant de 1 à 15 % en poids par rapport au poids total de la feuille.

4. La feuille de la revendication 1, où le mélange homogène d'enzymes comprend au moins une enzyme protéase, au moins une enzyme amylase, au moins une enzyme pectate-lyase, au moins une enzyme mannanase, au moins une enzyme lipase, ou une combinaison de celles-ci.

5. La feuille de n'importe lesquelles des revendications précédentes, la feuille ayant un poids allant d'environ 1 gramme à 8 grammes, cet intervalle étant inclusif.

6. La feuille de n'importe lesquelles des revendications précédentes, la feuille ayant une épaisseur allant d'environ 0,1 à 0,3 millimètre, cet intervalle étant inclusif.

7. La feuille de la revendication 2, où le substrat de nanocellulose comprend une fibre de nanocellulose qui est naturellement dérivée de bois résineux, de bois feuillu, de coques de soja, de paille de blé, de bagasse, de pulpe de betterave sucrière, ou d'une combinaison de ceux-ci.

8. La feuille de n'importe lesquelles des revendications précédentes, où l'ingrédient actif comprend un élément parmi un produit de nettoyage, un détergent pour la lessive, un agent tensioactif, un savon pour lave-vaisselle, un savon pour les mains, un nettoyant pour la peau ou un démaquillant, un adoucissant de tissus, une composition agricole, une composition dermatologique, une composition pharmaceutique, une composition cosmétique, ou une combinaison de n'importe lesquels des éléments précédents.

9. La feuille de n'importe laquelle des revendications précédentes, où l'ingrédient actif comprend :
au moins un élément parmi des renforçateurs de senteur, des réducteurs d'électricité statique, des agents d'élimination de taches, des agents d'antiredéposition, des agents de dégraissage, des agents javellisants, des agents de blanchiment, des agents de blanchiment fluorescents, des azurants, des agents d'inhibition de dégorgement, des capteurs de chlore, des agents chélateurs, des inhibiteurs de corrosion, des opacifiants, des suppresseurs de mousse, des renforçateurs de mousse, des agents de traitement, des colorants, des teintures, des polymères exhausteurs de tissus, des ajusteurs de pH, des agents tampons, des antibactériens, des antimicrobiens, des agents antiodeurs, et des parfums.

10. La feuille de n'importe laquelle des revendications précédentes, où l'ingrédient actif comprend :
au moins un élément parmi un shampooing, un après-shampooing, un nettoyant corporel, un nettoyant pour le visage, des hydratants, un démaquillant, un déodorant, des assainisseurs, des antibactériens, un écran solaire, et des ingrédients pour le toilettage d'animaux domestiques.

11. Un procédé de fabrication d'une feuille telle que définie dans la revendication 1, comprenant :
(i) le mélangeage du substrat de nanocellulose, de la version chimiquement modifiée de cellulose, et de la composition d'ingrédients actifs ensemble, produisant de ce fait une formulation de précurseur liquide ; et
(ii) le séchage de la formulation de précurseur liquide, produisant de ce fait une composition de matrice soluble, l'étape de séchage réduisant la teneur en eau de la formulation de précurseur liquide d'au moins 35 %.

12. Un procédé de fabrication d'une feuille telle que définie dans la revendication 1 comprenant :
(i) le mélangeage du substrat de nanocellulose, de la version chimiquement modifiée de cellulose, et de la composition d'ingrédients actifs ensemble, produisant de ce fait une formulation de précurseur liquide ;
(ii) le traitement de la formulation de précurseur liquide par une machine à enduire au rouleau sur une feuille intercalaire ;
(iii) le séchage de la formulation liquide en faisant passer la feuille intercalaire par un dispositif de chauffage pour former un rouleau de feuille séchée ; et
(iv) le découpage du rouleau de feuille séchée en feuilles individuelles.

13. Le procédé de la revendication 12, où le dispositif de chauffage est une étuve à convection.

14. Le procédé de soit la revendication 12 soit la revendication 13, où la feuille intercalaire passe à travers le dispositif de chauffage à une vitesse allant d'environ 0,5 mètre par minute à 1,5 mètre par minute.

15. Le procédé de n'importe lesquelles des revendications 12 à 14, où la formulation liquide n'est pas chauffée au-dessus de 49 °C (120 °F) et la température interne maximale du rouleau de feuille n'excède pas 49 °C (120 °F).
